# EUROPÄISCHE PATENTSCHRIFT

(11) **EP 4 115 863 B1**
(45) Veröffentlichungstag und Bekanntmachung des Hinweises auf die Patenterteilung: **28.10.2026**
(21) Anmeldenummer: 22178623.9
(22) Anmeldetag: 13.06.2022
(51) Int. Cl.: A61F 13/58, A44B 18/00, A61F 13/62

(54) **VERSCHLUSSBAND, HALBZEUGBANDMATERIAL UND VERFAHREN ZUR ABSTIMMUNG DER KLEBEKRÄFTE BEI EINEM VERSCHLUSSBAND**
SEALING TAPE, SEMI-FINISHED TAPE MATERIAL AND METHOD FOR ADJUSTING THE ADHESION FORCES OF A CLOSURE TAPE
BANDE DE FERMETURE, MATIÈRE EN BANDE DE DEMI-PRODUIT ET PROCÉDÉ DE RÉGLAGE DES FORCES D'ADHÉRENCE DANS UNE BANDE DE FERMETURE

(30) Priorität: 08.07.2021 DE 102021117720; 23.08.2021 DE 102021121816
(43) Veröffentlichungstag der Anmeldung: 11.01.2023
(73) Patentinhaber: Lohmann-koester GmbH & Co. KG, 96146 Altendorf (DE)
(72) Erfinder: NEUGEBAUER, Robert, 96146 Altendorf (DE)
(74) Vertreter: Reuther, Martin

(56) Entgegenhaltungen:
- EP-B1- 1 725 202
- JP-A- 2010 131 104

## Beschreibung

Die Erfindung betrifft ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst. Auch betrifft die Erfindung ein Halbzeugbandmaterial mit einer Maschinenrichtung und einer senkrecht hierzu angeordneten Querrichtung zur Herstellung eines derartigen Verschlussbands mittels Trennen des Halbzeugbandmaterials in Querrichtung, wobei die Maschinenrichtung der Höhe und die Querrichtung der Breite des Verschlussbands entsprechen, und ein Verfahren zur Abstimmung der Klebekräfte bei einem entsprechenden Verschlussband.

Derartige Verschlussbänder sind beispielsweise aus der WO 2019/207529 A1, der EP 0 755 665 A1, der EP 0951 888 A2, der WO 2005/000180 A1, der WO 2005/000181 A1, der EP 1 838 178 B1 und der WO 2005/074852 A1 bekannt.

Weiterer Stand der Technik ist aus EP1725202 bekannt.

Es ist Aufgabe vorliegender Erfindung gattungsgemäße Verschlussbänder, Halbzeugbandmaterialien bzw. Abstimmverfahren bereitzustellen, welche auf baulich einfache Weise die Verschlusskraft des jeweiligen Verschlussbands, mit welcher dieses wirken kann, maximieren.

Die Aufgabe der Erfindung wird durch Verschlussbänder, Halbzeugbandmaterialien bzw. Abstimmverfahren mit den Merkmalen der unabhängigen Ansprüche gelöst. Weitere, ggf. auch unabhängig hiervon, vorteilhafte Ausgestaltungen finden sich in den Unteransprüchen sowie der nachfolgenden Beschreibung.

Hierbei geht die Erfindung von der Grunderkenntnis aus, dass bei Verschlussbändern vor allem am Rand einer Flächenkomponente eines zweikomponentigen mechanischen Flächenverschlusssystems, wie beispielsweise eines Haken-Schlaufen-Verschlusssystems, baulich einfach Maßnahmen durchgeführt werden können, um die Verschlusskraft zu maximieren. Insbesondere an einem Rand, der einem Anfassenden, also einer Person, welche das Verschlussband anfasst bzw. bedient, bzw. einer Öffnungsvorzugsrichtung abgewandt ist, können geeignete Maßnahmen vorgesehen sein. Hier kann einerseits der entsprechende Randbereich des Flächenkomponentenrands geeignet ausgeführt sein, um die Flexibilität und die Gesamtfläche des Flächenverschlusses zu erhöhen oder andererseits in geeigneter Weise in der Nähe des Flächenkomponentenrands eine Klebfläche angeordnet sein. Insbesondere die Kombination dieser beiden aus der Grunderkenntnis folgenden Ansätze ist vorteilhaft. Insbesondere kommt mithin von der Flächenkomponente ausgehend dem in Richtung der Öffnungsvorzugsrichtung liegenden Randbereich und dessen Gestaltung eine besondere Bedeutung zu, indem hier am Rand der Flächenkomponente eine möglichst hohe Variation des Rands bereitgestellt ist bzw. sichergestellt ist, dass von dort ausgehend in Öffnungsvorzugsrichtung nur noch Klebefläche zu finden ist.

So ermöglicht die Ausgestaltung des entsprechenden Randbereichs mit einer ausreichend großen Variation, also mit einer ausreichend großen Veränderung senkrecht zu einer Randbereichsmittenlinie, eine hohe Flexibilität des Hakenmaterials, ohne dass die bauliche Einheit desselben zwingend zerstört werden muss, wie dieses beispielsweise bei parallel zu der Höhe verlaufenden, geteilten Hakenstreifen nach dem Stand der Technik der Fall ist.

Andererseits ermöglicht die Tatsache, dass von jedem Flächenpunkt der Klebefläche in Richtung einer Öffnungsvorzugsrichtung ausgehend kein Flächenpunkt der ersten Flächenkomponente angeordnet ist, dass die Gefahr, dass die Klebefläche Material mitnimmt und dieses dann die Haken überdeckt oder sonstwie kontaminiert und somit eine Verschlechterung der Verschlusskraft bedingt, minimiert werden kann.

Um gattungsgemäße Verschlussbänder bereitzustellen, welche auf baulich einfache Weise die Verschlusskraft des jeweiligen Verschlussbands, mit welcher dieses wirken kann, maximieren, kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Seite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittellinie, die Schnittpunkte zwischen den Flächenkomponentenrand und der Randbereichsmittellinie trägt, variiert, wobei die Variation in Richtung der Höhe Berge und Täler aufweist und wobei der Variation in dem variierenden Randbereich ein Mittenvariationswert zugeordnet werden kann, welcher von der absoluten Abweichung von Variationsspannen zwischen Berge und Täler zu der Randbereichsmittenlinie abhängig ist, und wobei der Mittenvariationswert zumindest so groß wie die Hälfte des mittleren Abstandes parallel zur Höhe zwischen zwei benachbarten Bergmitten ist.

Kumulativ bzw. alternativ hierzu, kann sich ein Verschlussband mit einer Befestigungsseite, an welcher eine erste Flächenkomponente eines zweikomponentigen mechanischen Verschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Bergen und Täler aufweist und wobei die Variation in dem variierenden Randbereich ein Mittenvariationswert zugeordnet werden kann, welcher von der absoluten Abweichung von Variationsspannen zwischen Berge und Täler zu der Randbereichsmittenlinie abhängig ist, und wobei der Mittenvariationswert zumindest so groß wie die Hälfte des mittleren Abstandes parallel zur Höhe zwischen zwei benachbarten Talmitten ist, um Verschlussbänder bereitzustellen, welche auf baulich einfache Weise die Verschlusskraft des jeweiligen Verschlussbands, mit welcher dieses wirken kann, maximieren.

Durch eine derartige Ausgestaltung der Berge bzw. Täler kann eine ausreichend große Variation bzw. Veränderung, wie sie vorstehend als Teil der Grundidee gefordert ist, gewährleistet werden.

Der Begriff "Verschlussband" beschreibt im vorliegenden Zusammenhang vorzugsweise ein Band aus meist kunststoffartigen Materialien, welches dazu dient, etwas zu verschließen. Hierbei kann das Verschlussband für sämtliche Objekte verwendet werden, bei welchen das Objekt in irgendeiner Form verschlossen werden kann. Beispielsweise könnte das Verschlussband für Hygieneartikel, wie Windeln, zum Einsatz kommen, um diese zu verschließen. Das Verschlussband könnte jedoch auch beispielsweise bei textilen Objekten, wie Kleidung zum Einsatz kommen, um beispielsweise eine Jacke zu verschließen.

Vorzugsweise ist eine Seite des Verschlussbandes als Befestigungsseite ausgebildet, welche letztendlich der Befestigung für den eigentlichen durch das Verschlussband bereitgestellten Verschlusses dient.

Vorteilhafterweise ist an dieser Befestigungsseite eine erste Flächenkomponente eines zweikomponentigen mechanischen Verschlusssystems befindlich. Unter einem "zweikomponentigen mechanischen Flächenverschlusssystem" kann im vorliegenden Zusammenhang beispielsweise ein Haken-Schlaufen-System verstanden werden, sodass eine erste Flächenkomponente beispielsweise Hakenelemente darstellen können, welche für sich alleine noch kein mechanisches Flächenverschlusssystem bilden können. Üblicherweise benötigt ein zweikomponentiges mechanisches Flächenverschlusssystem zwei Komponenten, die nur im Zusammenhang bzw. in Wechselwirkung miteinander gemeinsam ein zweikomponentiges mechanisches Flächenverschlusssystem bilden können. Dementsprechend können bei einem Haken-Schlaufen-System die Hakenelemente dann in entsprechende Schlaufenelemente, die beispielsweise durch eine Schlaufen aufweisende textile Lage oder durch ein ausreichend Schlaufen oder zu hintergreifende Fasern aufweisendes NonWoven bereitgestellt sind und dementsprechend ebenfalls flächig angeordnet sind, eingreifen und somit einen lösbaren Verschluss ermöglichen. Es versteht sich, dass, je nach Sichtweise, bei einer derartigen Ausgestaltung die Hakenelemente oder die Schlaufenelemente in ihrem jeweiligen Verbund als erste Flächenkomponente bzw. als zweite Flächenkomponente des entsprechenden zweikomponentigen mechanischen Verschlusssystems bezeichnet werden können. Je nach konkreter Umsetzung kann das zweikomponentige mechanische Verschlusssystem auch durch andere flächige Komponenten, wie Haken-Ösen-Anordnungen oder flächige Druckknopfanordnungen, ausgebildet werden. Je nach konkreter Ausgestaltung können die beiden Komponenten des zweikomponentigen mechanischen Flächenverschlusssystems auch gleich bzw. identisch ausgebildet sein, solange eine ausreichende mechanische Verbindung zwischen denselben ausgebildet werden kann. Insoweit zwei flächig ausgebildete Komponenten vorgesehen sind, die miteinander mechanisch verbunden und vorzugsweise auch wieder gelöst werden können, kann von "zweikomponentigen mechanischen Flächenverschlusssystemen gesprochen werden. Zu unterscheiden sind derartige zweikomponentige mechanische Flächenverschlusssysteme zum einen von Klebeflächen, die lediglich einkomponentige Verschlusssysteme darstellen, wenn keine besondere ausgebildete Gegenflächen bzw. Landeelemente vorgesehen sein müssen, während derartige Klebeflächen, wenn besondere Landeflächen für die vorgesehen sind, zwar als zweikomponentig bezeichnet werden können, jedoch nicht mechanisch eine Verbindung bewirken. Zum anderen sind derartige zweikomponentige mechanische Flächenverschlusssysteme von punktuellen mechanischen Verschlusssystemen zu unterscheiden, bei denen lediglich zwei singuläre Komponenten, wie ein Knopf und ein Knopfloch, wie ein Haken und eine Schlaufe oder Öse, einzelne Druckknöpfe, miteinander verbunden werden können.

Das Verschlussband ist vorzugsweise als sehr flaches Element ausgestaltet, welches neben einer Vorderseite auch eine Rückseite sowie eine schmale umlaufende Kante aufweist.

Im vorliegenden Zusammenhang beschreibt die "Breite" vorzugsweise eine Erstreckung parallel zu der Befestigungsseite und zu der Rückseite. Entsprechend kann unter der "Höhe" die Erstreckung des Verschlussbandes senkrecht zur Breite verstanden werden. An sich ist es mithin lediglich eine Frage der Definition, welche Erstreckungsrichtung des Verschlussbandes als Breite und welche dann als Höhe bezeichnet wird. Andererseits werden derartige Verschlussbänder häufig aus einem sehr langen Halbzeug bereitgestellt, welches sich in einer Maschinenrichtung, also in einer Richtung, in welcher das Halbzeug bei seiner Herstellung eine Maschine durchläuft, und eine senkrecht hierzu angeordnete Querrichtung erstreckt. Durch Trennen in Querrichtung werden dann die Verschlussbänder bereitgestellt, wobei dann üblicherweise die Querrichtung die Breite des Verschlussbandes und die Maschinenrichtung die Höhe des Verschlussbands definiert. In der Regel wird dann auch die Querrichtung bzw. die Breite die Richtung darstellen, in welcher das Verschlussband Kräfte aufnimmt und seine verschließende Wirkung ausübt bzw. parallel zu welcher eine Öffnungsvorzugsrichtung zum Öffnen des durch das Verschlussband bereitgestellten Verschlusses zu finden ist.

Die "Verschlussbandumgrenzung" beschreibt dementsprechend vorzugsweise die Kante, die um das gesamte Verschlussband umläuft und diese somit umgrenzt. Die Verschlussbandumgrenzung kann somit die Geometrie des Verschlussbandes beschreiben und wird in der Regel durch Schnitte oder ähnliche Durchtrennungen bereitgestellt.

Die Verschlussbandumgrenzung setzt sich in der Regel aus verschiedenen Verschlussbandgrenzen zusammen, wobei im vorliegenden Zusammenhang vorzugsweise zwei Verschlussbandgrenzen parallel zur Breite erstreckt sind und sich zwei weitere Verschlussbandgrenzen senkrecht zur Breite erstrecken. Durch diese Anordnung kann eine vorteilhafte rechteckige Grundform des Verschlussbandes erzielt werden. Hierbei schließen also die Verschlussbandgrenzen mit den Ecken einer rechteckigen Grundform des Verschlussbandes ab. Alle Verschlussbandgrenzen bilden gemeinsam die Verschlussbandumgrenzung.

Es versteht sich, dass die Verschlussbandgrenzen nicht zwingend geradlinig ausgebildet sein müssen, so dass beispielsweise auch gewellte oder gebogene Verschlussbandgrenzen vorgesehen sein können, solange diese eine Richtungskomponente in die vorgesehene Richtung aufweisen. Auch die Ausrichtung senkrecht oder parallel zur Breite kann dementsprechend bereits durch eine entsprechende Richtungskomponente umgesetzt sein. Ebenso müssen dementsprechend nicht unbedingt Verschlussbandgrenzen genau senkrecht zu einander oder genau parallel zueinander ausgerichtet sein. Vielmehr reicht auch hier eine Richtungskomponente bzw. aus, um eine entsprechende Bedingung zu erfüllen.

Unter dem "Flächenkomponentenrand" kann in vorliegenden Zusammenhang vorzugsweise der gesamte Rand der entsprechenden Flächenkomponente verstanden werden, der also die Flächenkomponente umgrenzt und somit die räumliche bzw. geometrische Ausgestaltung der Flächenkomponente beschreibt. Der Bereich innerhalb des Flächenkomponentenrandes bildet die Flächenkomponente, sodass unterschiedliche Anordnungen des Flächenkomponentenrandes unterschiedliche Ausgestaltungen der Flächenkomponente vorgeben.

Auch der Rand einer Flächenkomponente, also der Flächenkomponentenrand kann in Bereich des Rands bzw. des Flächenkomponentenrands, also in Randbereiche unterteilt werden. Insbesondere kann, da es sich bei der Flächenkomponente um eine flächiges Gebilde handelt, welches entlang des Verschlussbands ausgerichtet ist, kann der Flächenkomponentenrand in Bereiche unterteilt werden, von denen sich welche - ähnlich der Verschlussbandgrenzen -im Wesentlichen senkrecht zur Breite und somit parallel zur Höhe oder parallel zur Breite und somit senkrecht zur Höhe erstrecken. Häufig wird die Flächenkomponente entsprechend des Trennens in Querrichtung bzw. parallel zur Breite auch parallel zur Breite geradlinig ausgerichtete Randbereiche aufweisen, zwischen denen dann konsequenterweise in der Höhe verlaufende bzw. parallel zur Höhe verlaufende Bereiche definiert werden können. Bei einem komplexer ausgebildeten Flächenkomponentenrand, bei welchem in Bezug auf die durch die Verschlussbandbegrenzung ergebenden Ecken korrespondierende Ecken des Flächenkomponentenrands vorliegen, kann dann beispielsweise die Flächenkomponente zwischen diesen korrespondierenden Ecken in Bereiche, die in der Höhe bzw. parallel zur Höhe verlaufen, und Bereiche, die in der Breite bzw. parallel zur Breite verlaufen, unterteilt werden. Sollten derartige Ecken nicht klar definiert werden, so können, um unterschiedliche Randbereiche und insbesondere dann auch einen in der Höhe verlaufenden Randbereich zu definieren, entsprechende Flächenkomponenten in vom Zentrum oder der Mitte der Flächenkomponente ausgehende Sektoren unterteilt werden, deren Sektorengrenzen zu den durch die Verschlussbandbegrenzung definierte Ecken laufen oder deren Sektorengrenzen jeweils in Winkeln von 90° zueinander und in Winkeln von 45° zu der Breite und zu der Höhe stehen. Auf diese Weise lässt bei nahezu allen Formen, in welchen die Flächenkomponente ausgebildet sein könnte, eine eindeutige und nachvollziehbare Unterteilung in Randbereiche, die in der Höhe, also parallel zu Höhe verlaufen bzw. die in der Breite, also parallel zur Breite verlaufen, vornehmen.

Der "variierende Randbereich" beschreibt vorzugsweise, dass zumindest ein Randbereich des Flächenkomponentenrandes, also ein Teilbereich desselben, nicht gerade ausgebildet ist, sondern irgendwie in seinem Verlauf variiert, also beispielsweise kurvenartig, zick-zack-artig oder wellenartig, ausgestaltet ist.

Unter der "Variation" eines Randbereichs kann vorzugsweise verstanden werden, dass dieser in seinem Verlauf, beispielsweise entlang der Höhe des Verschlussbands, sich regelmäßig oder unregelmäßig senkrecht zu einer Randbereichsmittenlinie verändert, also variiert, sodass der variierende Randbereich über seinen Verlauf vorzugsweise mehrere Vorzeichenwechsel in seiner Steigung aufweist. Vorzugsweise durch diese Richtungswechsel entstehen dann in dem variierenden Randbereich Berge und Täler, was genau durch einen derartigen Vorzeichenwechsel bei der Steigung bzw. durch ein Maximum oder Minimum im mathematischen Sinne definiert sein kann. Dieses bedeutet, dass dann in einem Tal oder Berg jeweils wenigstens ein Punkt definiert ist, bei welchem die erste Ableitung Null und die zweite Ableitung ungleich Null ist. Insoweit es sich vorliegend bei den Flächenkomponenten um materielle Gegenstände handelt, ist an sich ausgeschlossen, dass in der Steigung bzw. Ableitung tatsächlich Unstetigkeiten zu finden sind, da die entsprechenden Übergänge und Richtungswechsel nicht auf atomare Ebene abrupt realisierbar sein sollten.

Unter den "Bergen" können vorzugsweise die Abschnitte des variierenden Randbereichs mit einem Vorzeichenwechsel der Steigung vom positiven zum negativen Verstanden werden, während bei "Tälern" vorzugsweise der Abschnitt mit einem Vorzeichenwechsel der Steigung des variierenden Randbereichs vom negativen zum positiven verstanden werden kann. Dieses entspricht auch der allgemeinen Vorstellung, dass zum Erklimmen eines Berges zunächst eine positive Steigung und dann, bei Herbsteigen, eine negative Steigung zu bewältigen ist, was insbesondere auch unabhängig von der Richtung erfolgt, in welcher ein Berg überwunden wird, während dieses beim Durchqueren eines Tals genau umgekehrt ist, da zunächst in das Tal mit einer negativen Steigung herabgestiegen werden muss, um dann aus dem Tal heraus mit einer positiven Steigung zu gelangen. Ein rein mathematischer Ansatz kann, unter Berücksichtigung der Bewegungsrichtung, auf die erste Ableitung gerichtet sein, welche unter Berücksichtigung der Bewegungsrichtung an einem Berg vom Positiven zum Negativen und durch ein Tal vom Negativen zum Positiven wechselt.

Jeder Berg weist zudem eine Bergmitte auf. Wenn der Fall vorliegt, dass der Berg im Bereich seiner höchsten Stelle gekrümmt ist, ist die Bergmitte dann das Maximum bzw. ein Maximum des variierenden Randbereichs. Bei anderen Ausgestaltungen des Berges kann dann der Berg eine über eine Strecke geradlinig ausgebildete Bergspitze aufweisen, so dass dann die Bergmitte der Mittelpunkt der die Bergspitze ausbildenden Strecke. Beispielsweise wäre bei einem sinusförmigen Verlauf des variierenden Randbereichs klar ein Maximum definiert, welches eine entsprechende Bergmitte darstellen kann. Ist jedoch kein eindeutiges Maximum durch eine Krümmung gegeben, sondern der Berg beispielsweise über gerade Strecken gebildet, bildet der Mittelpunkt einer dieser die Berg ausbildenden Strecken dann die Bergmitte.

Dementsprechend kann auch einem Tal eine Talmitte zugeordnet werden. Ähnlich wie bei den Bergmitten, kann im vorliegenden Zusammenhang die Talmitte als das zugehörige Minimum des variierenden Randbereichs verstanden werden, wenn das Tal in seinem tiefsten Bereich gekrümmt ist. Sollte dies nicht der Fall sein, bildet der Mittelpunkt der das Tal ausbildenden Strecke entsprechend die Talmitte.

Ein vollständiger Berg beginnt vorzugsweise jeweils am tiefsten Punkt, also dem Minimum, seiner benachbarten beiden Täler, während entsprechend ein vollständiges Tal vorzugsweise jeweils am höchsten Punkt, also dem Maximum, der das Tal einschließenden Berge beginnt. Insoweit es sich bei einem zugehörigen Minimum um eine Strecke handelt, beginnt ein Berg vorzugsweise jeweils an den Talmitten der benachbarten Täler oder, alternativ, an dem Punkt der jeweiligen Strecken, die dem Berg am nächsten sind. Dementsprechend beginnt ein Tal vorzugsweise jeweils an den Bergmitten der benachbarten Berge oder, alternativ, an dem Punkt der jeweiligen Strecken, die dem Tal am nächsten sind, wenn es sich bei einem zugehörigen Maximum um eine Strecke handelt.

Die bereits vorstehend erläuterten Randbereichsmittenlinien können zunächst auf sehr verschieden Weise definiert werden, um dennoch eine klare und eindeutige Festlegung der Parameter, wie den Mittenvariationswert oder einer Variationsspanne, zu ermöglichen. Allgemein kann jede Linie, welche durch den jeweiligen Randbereich gelegt werden kann und den Flächenkomponentenrand an mehreren Schnittpunkten schneidet, als Randbereichsmittenlinie bezeichnet werden. Insbesondere handelt es sich hierbei um eine Gerade, was insbesondere Berechnungen erleichtert und nachvollziehbar belässt.

Vorzugsweise kann die Randbereichsmittellinie als Mittelwert des variierenden Randbereichs definiert sein, wobei letztlich diesbezüglich sämtlich Arten, entsprechende Mittelwerte zu berechnen, zur Anwendung kommen können. Andererseits ist es denkbar, die Randbereichsmittenlinie beispielsweise als Schwerpunktslinie der Berge bzw. der Täler zu definieren. Ebenso kann die Randbereichsmittenlinie ggf. als eine Linie, die von einem Ende des Randbereichs bis zum anderen Ende des Randbereichs reicht, definiert sein.

Vorzugsweise ist die jeweilige Randbereichsmittenlinie rml(x) bzw. bei diskreten Messpunkten xₘ die jeweilige Randbereichsmittenlinie rml(xₘ) jedoch, wie vorstehend bereits angedeutet, aus dem Mittelwert <r> des variierenden Randbereichs r(xₘ) bzw. r(x) entlang des Verlaufs x dieser Randbereichsmittenlinie, also insbesondere über der Höhe bzw. senkrecht zur Breite, bestimmt.

Der Mittelwert orientiert sich an der Mittelwertberechnung, wie sie auch bei Rauheitsuntersuchungen angewandt wird. Hierbei lässt sich der Mittelwert <z> durch die Formel $\left〈z\right〉 = \frac{1}{MN} {\sum }_{m = 1}^{M} {\sum }_{n = 1}^{N} z \left({x}_{m}, {y}_{n}\right)$ (1) berechnen, wobei M und N die Zahl der jeweiligen Messpunkte in der Ebene, in welcher die Rauheit bestimmt werden soll, xₘ und yₙ die Raumkoordinaten in dieser Ebene mit m und n als Zähler sowie z die jeweilige Höhe der Ebene an den zugehörigen Raumkoordinaten xₘ, yₙ sind. Dementsprechend folgt dann für die Randbereichsmittenlinie rml(x) mit $rml \left(x\right) = \left〈r\right〉$ (2) aus dem entsprechenden Mittelwert <r>, wobei links der funktionale Zusammenhang bei kontinuierlichen Funktionen dargestellt ist, falls der jeweils zu untersuchende Randbereich r(x) nicht in Form diskreter Punkte, sondern in Form eines funktionalen Zusammenhangs bekannt ist, was beispielsweise durch eine ausreichend genaue funktionale Annäherung bzw. durch ein entsprechenden funktionalen Fit zwischen einem Anfang a und einem Ende e des zu untersuchenden Randbereichs r(x) umgesetzt werden kann: $rml \left({x}_{m}\right) = \left〈r\right〉 = \frac{1}{M} {\sum }_{m = 1}^{M} r \left({x}_{m}\right) \approx rml \left(x\right) = \left〈r\right〉 = \frac{1}{e - a} {\int }_{a}^{e} r \left(x\right) dx$ (3)

Es ist hierbei zu berücksichtigen, dass an sich die Randbereichsmittenlinie rml bei diskreten Messpunkten xₘ lediglich für die entsprechenden Messpunkte xₘ relevant erscheint, wobei dieses, da <r> als Mittelwert einen konstanten Wert darstellt, in diesem konkreten Fall unerheblich erscheint. Bei abweichenden Definitionen der Randbereichsmittenlinie könnten hier jedoch Unterschiede bestehen, beispielsweis wenn die Randbereichsmittenlinie lokalen Abweichungen unterliegt, was beispielsweise bei einer Variation, welche aus zwei Variationen mit unterschiedlichen Frequenzen besteht, sinnvoll erscheinen könnte, wie dieses bei Rauheitsuntersuchungen an gekrümmten Oberflächen ebenfalls vorgenommen wird. Andererseits erleichtert eine Beschränkung auf eine konstante bzw. geradlinige Randbereichsmittenlinie in vorliegendem Zusammenhang angesichts der zu erwartenden Dimensionen und Messgenauigkeiten ausreichend und vorteilhaft.

Insbesondere kann auch eine Variationsspanne v(xₘ) bzw. v(x) derart definiert werden, dass diese jeweils als der Abstand zwischen Randbereichsmittenlinie rml(xₘ) bzw. rml(x) und dem variierenden Randbereich r(xₘ) bzw. r(x) bestimmt ist: $v \left({x}_{m}\right) = v \left({x}_{m}\right) - rml \left({x}_{m}\right) \approx v \left(x\right) = v \left(x\right) - rml \left(x\right)$ (4) Die Variationsspanne v(xₘ) bzw. v(x) ist mit dem Höhenwert aus dem Bereich der Rauheitsuntersuchungen zu vergleichen. Somit kann zu jedem beliebigen Punkt des variierenden Randbereichs, insbesondere jedoch zu jedem Messpunkt, eine Variationsspanne ermittelt werden, sodass es im variierenden Randbereich bei einer kontinuierlichen Funktion letztlich unendlich viele Variationsspannen gibt. Je nach konkreter Umsetzung kann jedoch eine hiervon abweichende Definition der Variationsspannen erfolgen. So können diese Beispielsweise auch von einer definierten Grundlinie oder in einem vektoriellen Raum bestimmt werden. Letztlich ist jede Größe, die als lokales Maß für die Variation des variierenden Randbereichs dienen kann, als Variationsspanne geeignet. Andererseits erscheint die vorstehend über die Randbereichsmittenlinie definierte Variationsspanne in der Praxis gut anwendbar und ausreichend.

Als Mittenvariationswert kann letztlich jede von dem Absolutwert der Variationsspannen abhängige Größe genutzt werden. Vorzugsweise wird eine Größe gewählt, die entsprechend Aussagen über einen Mittelwert der Variationsspannen geben kann, wobei die Art des Mittelwerts der jeweiligen Art der Variation entsprechend angepasst werden kann, wie insbesondere bei anderen Mittelwertsberechnungen und -untersuchungen bereits hinlänglich bekannt. Insbesondere kann die Wahl der Größe für den Mittenvariationswert auch von der Wahl der Bestimmungsart für die Randbereichsmittenlinie bzw. der Variationsspanne abhängig gemacht werden, um hier kompatible Berechnungen durchführen zu können.

Es ist insbesondere von Vorteil, wenn ein Mittenvariationswert der Mittelwert der absoluten Variationsspannen über der Erstreckung des variierenden Randbereichs ist. Auch hierdurch kann der Begriff des Mittenvariationswertes klar und eindeutig definiert werden.

Der Mittenvariationswert Mₐ orientiert sich dann an den Mittenrauwert Rₐ, der einem Fachmann allgemein aus den Rauheitsuntersuchungen bekannt ist. Der Mittenrauwert, dargestellt durch das Symbol Rₐ, gibt den mittleren Abstand eines Messpunktes - auf der Oberfläche - zur Mittellinie <z> (siehe oben Formel (1)) an. Die Mittellinie schneidet innerhalb der Bezugsstrecke das wirkliche Profil so, dass die Summe der Profilabweichungen in einer parallelen Ebene zur Mittellinie <z> auf die Länge der Messstrecke verteilt wird. Der Mittenrauwert Rₐ entspricht also dem arithmetischen Mittel der betragsmäßigen Abweichung von der Mittellinie <z>. In zwei Dimensionen berechnet sie sich aus: ${R}_{a} = \frac{1}{MN} {\sum }_{m = 1}^{M} {\sum }_{n = 1}^{N} \left|z\left({x}_{m}, {y}_{n}\right)-\left〈z\right〉\right|$ (5)

Dementsprechend ergibt sich dann unter Berücksichtigung der Formeln (2) bis (4) für den auf diese Weise definierten Mittenvariationswert Mₐ: ${M}_{a} = \frac{1}{M} {\sum }_{m = 1}^{M} \left|v\left({x}_{m}\right)-\left〈r\right〉\right| \approx {M}_{a} = \frac{1}{e - a} {\int }_{a}^{e} \left|v\left(x\right)-\left〈r\right〉\right| dx$ (6) mit den bereits vorstehend definierten Parametern bzw. ${M}_{a} = \frac{1}{M} {\sum }_{m = 1}^{M} \left|r\left({x}_{m}\right)-rml\left({x}_{m}\right)\right| \approx {M}_{a} = \frac{1}{e - a} {\int }_{a}^{e} \left|r\left(x\right)-rml\left(x\right)\right| dx$ (7) oder ${M}_{a} = \frac{1}{M} {\sum }_{m = 1}^{M} \left|v\left({x}_{m}\right)\right| \approx {M}_{a} = \frac{1}{e - a} {\int }_{a}^{e} \left|v\left(x\right)\right| dx$ (8) zum Erhalt eines Maßes, nach welchem die Variation objektiv in ihrem Ausmaß bewertet und verglichen werden kann.
Dementsprechend ist dann der Variation in dem variierenden Randbereich ein Mittenvariationswert zuordenbar, der dann auch mit dem mittleren Abstand zwischen zwei Bergmitten oder Talmitten verglichen werden kann.

Entsprechend den verschiedenen Arten der Rauheitsbestimmungen, beispielsweise einem Mittenrauwert, einer quadratischen Rauheit oder einer gemittelten Rautiefe, können hier, je nach konkret vorliegender Variation, ggf. geeignetere Größen aus der Variation bzw. aus dem Verlauf des variierenden Randbereichs gewählt und entsprechend für die Bestimmung der Qualität der Variation genutzt werden. Dieses ermöglicht dann eine entsprechende Zuordnung eines Mittenvariationswerts, der für den Vergleich mit dem mittleren Abstand zwischen zwei Bergmitten oder Talmitten genutzt werden kann.

Es ist vorteilhaft, wenn der Mittenvariationswert zumindest so groß wie der 0,38-fache mittlere Abstand parallel zur Höhe zwischen zwei benachbarten Bergmitten ist. Je höher dieser Unterschied ist, desto größer sind auch die Erstreckung der ersten Flächenkomponente und somit auch die mögliche Verschlusskraft. Außerdem ist eine hohe Flexibilität entlang der Höhe gegeben, was dementsprechend die Verschlusskraft weiter erhöht.

Kumulativ bzw. alternativ hierzu, um dieselben Vorteile zu erzielen, kann der Mittenvariationswert dementsprechend zumindest so groß wie der 0,38-fache mittlere Abstand parallel zur Höhe zwischen zwei benachbarten Talmitten sein.

Entsprechend kann der Mittenvariationswert vorzugsweise zumindest so groß wie der 0,39-fache mittlere Abstand parallel zur Höhe zwischen zwei benachbarten Bergmitten bzw. zwischen zwei benachbarten Talmitten sein.

Es hat sich gezeigt, dass insbesondere, wenn der Mittenvariationswert zumindest so groß wie der 0,4-fache mittlere Abstand parallel zur Höhe zwischen zwei benachbarten Bergmitten bzw. zwei benachbarten Talmitten ist, sehr gute Ergebnisse hinsichtlich der Verschlusskraft bei hoher Flexibilität erzielt werden können.

Um Verschlussbänder bereitzustellen, welche auf baulich einfach Weise die Verschlusskraft des jeweiligen Verschlussbands, mit welcher dieses wirken kann, maximieren, kann sich kumulativ bzw. alternativ hierzu ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei die Zahl der Berge 2 pro 1 cm Erstreckung des Randbereichs übersteigt.

Um denselben Vorteil zu erzielen, kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, kumulativ bzw. alternativ zu den übrigen bereits genannten vorteilhaften Merkmalskombinationen, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei die Zahl der Täler 2 pro 1 cm Erstreckung des Randbereichs übersteigt.

Im vorliegenden Zusammenhang kann insbesondere die Zahl der Berge bzw. der Täler als 1 angerechnet werden, wenn bei einem Berg bzw. bei einem Tal die Vorzeichenumkehr der Steigung des variierenden Randbereichs bzw. der Variation abgeschlossen ist. Somit kann nur ein Wechsel der Variation von einer negativen Steigung zu einer positiven Steigung bzw. von einer positiven Steigung zu einer negativen Steigung als ein gesamter Berg bzw. ein gesamtes Tal angesehen werden. Möglicherweise ist auf dem Verschlussband nur ein Teil eines Berges bzw. eines Tals angeordnet, wenn beispielsweise bei der Trennung eines entsprechenden Halbzeugbandmaterials in Querrichtung auf die Phase der Variation keine Rücksicht genommen wird, sodass dann der Berg bzw. das Tal nicht als ein Ganzes angesehen werden kann. Beispielsweise ist es denkbar, dass auf 1 cm Erstreckung des Randbereichs ein Berg und ein möglicher Fuß eines zweiten Berges angeordnet ist. In einem solchen Fall wäre die Zahl der Berge jedoch 1 und somit unter 2 pro 1 cm Erstreckung des Randbereichs.

Hinsichtlich der möglichen Ausgestaltungen der Berge und Täler wird insbesondere auch auf die weiter vorstehenden Erläuterungen verwiesen.

Durch eine derartige Ausgestaltung der Berge bzw. Täler kann eine ausreichend große Variation bzw. Veränderung, wie sie vorstehend als Teil der Grundidee gefordert ist, gewährleistet werden.

Vorzugsweise übersteigt die Zahl der Berge 2,3 pro 1 cm Erstreckung des Randbereichs. Je höher diese Dichte ist, desto größer ist die Erstreckung der ersten Flächenkomponente und somit auch die mögliche Verschlusskraft, bei hoher Flexibilität entlang der Höhe, was dementsprechend die Verschlusskraft weiter erhöht. Dies gilt insbesondere bei einer ausreichend hohen Variationsspanne.

Jedoch sollte die Dichte nicht zu hoch gewählt werden, da sonst eine maschinelle Herstellung zu schwierig wird. Bei experimentellen Versuchen hat sich herausgestellt, dass die Zahl 5 pro 1 cm Erstreckung als ein sinnvolles Maximum erscheint.

Um die vorstehend genannten Vorteile zu erzielen, kann es kumulativ bzw. alternativ vorteilhaft sein, wenn die Zahl der Täler 2,3 pro 1 cm Erstreckung des Randbereichs übersteigt.

Besonders vorteilhaft ist eine Ausgestaltung, bei der die Zahl der Berge bzw. Täler 2,4 pro 1 cm Erstreckung des Randbereichs übersteigt.

Um eine entsprechend vorteilhafte Dichte für eine bessere Verschlusskraft bereitzustellen, kann auch die Zahl der Berge 2 pro 0,9 cm Erstreckung des Randbereichs übersteigen. Entsprechend vorteilhaft ist auch eine Ausgestaltung, bei der die Zahl der Täler 2 pro 0,9 cm Erstreckung des Randbereichs übersteigt.

Für eine hohe Verschlusskraft bei hoher Flexibilität entlang der Höhe kann die Zahl der Berge bzw. Täler 2 pro 0,8 cm Erstreckung des Randbereichs übersteigen.

Auch kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei die Summe aus dem Absolutwert der Variationsspanne einer Bergmitte und dem Absolutwert der Variationsspanne einer zu dieser Bergmitte benachbarten Talmitte zumindest so groß ist, wie der doppelte Abstand parallel zur Höhe zwischen dieser Talmitte und dieser Bergmitte, um Verschlussbänder bereitzustellen, welche auf baulich einfache Weise die Verschlusskraft des jeweiligen Verschlussbandes, mit welcher dieses wirken kann, maximieren.

Der "Absolutwert" kann im vorliegenden Zusammenhang als der Betrag der Variationsspanne verstanden werden. Es handelt sich also um den Wert der Zahl ohne Vorzeichen. Der Absolutwert der Variationsspanne ist somit immer ein positiver Wert.

Vorteilhafterweise ist die Summe aus dem Absolutwert der Variationsspanne einer Bergmitte und dem Absolutwert der Variationsspanne einer zu dieser Bergmitte benachbarten Talmitte zumindest 1,2 mal so groß, wie der doppelte Abstand parallel zur Höhe zwischen dieser Talmitte und dieser Bergmitte.

Besonders vorteilhaft ist hierbei, wenn die Summe aus dem Absolutwert der Variationsspanne einer Bergmitte und dem Absolutwert der Variationsspanne einer zu dieser Bergmitte benachbarten Talmitte zumindest 1,5 mal so groß ist, wie der doppelte Abstand parallel zur Höhe zwischen dieser Talmitte und dieser Bergmitte. Je höher dieser Wert ist, desto größer sind auch die Erstreckung der ersten Flächenkomponente und somit auch die mögliche Verschlusskraft.

Kumulativ bzw. alternativ hierzu, um Verschlussbänder bereitzustellen, welche auf baulich einfache Weise die Verschlusskraft des jeweiligen Verschlussbandes, mit welcher dieses wirken kann, maximieren, kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und sich ebenfalls parallel zur Befestigungsseite und zur Rückseite erstreckenden und senkrecht zur Breite erstreckenden und senkrecht zur Breite ausgerichteten Höhe sowie mit einer umlaufenden Verschlussbandumgrenzung, die zwei sich parallel zur Breite erstreckender Verschlussbandgrenzen und zwei sich senkrecht zur Breite erstreckender Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei die Summe aus dem Absolutwert der Variationsspanne einer Bergmitte und dem Absolutwert der Variationsspanne einer zu dieser Bergmitte benachbarten Talmitte zumindest so groß ist, wie die mittleren Abstände der Bergmitten und/oder die mittleren Abstände der Talmitten.

Im vorliegenden Zusammenhang kann unter den "mittleren Abständen" insbesondere verstanden werden, dass der Mittelwert aller Abstände berechnet wird. Hierbei können insbesondere alle Abstände aller benachbarten Bergmitten zueinander ermittelt und durch die Anzahl der ermittelten Abstände geteilt werden, sodass hierdurch der mittlere Abstand der Bergmitten folgen. Die mittleren Abstände der Talmitten ermitteln sich vorzugsweise entsprechend den mittleren Abständen der Bergmitten.

Insbesondere kann dementsprechend kumulativ bzw. alternativ hierzu die Summe aus dem Absolutwert der Variationsspanne einer Bergmitte und dem Absolutwert der Variationsspanne einer zu dieser Bergmitte benachbarten Talmitte zumindest 1,2 mal so groß, wie die mittleren Abstände der Bergmitten sein, um eine entsprechend hohe Verschlusskraft bei hoher Flexibilität zu erreichen. Bei einer entsprechend 1,5 mal so großen Ausgestaltung scheinen die Effekte besonders vorteilhaft zu sein.

Kumulativ bzw. alternativ hierzu, um eine entsprechend hohe Verschlusskraft bei hoher Flexibilität zu erreichen, kann die Summe aus dem Absolutwert der Variationsspanne einer Bergmitte und dem Absolutwert der Variationsspanne einer zu dieser Bergmitte benachbarten Talmitte zumindest 1,2mal so groß, wie die mittleren Abstände der Talmitten sein. Vorzugsweise ist die Summe aus dem Absolutwert der Variationsspanne einer Bergmitte und dem Absoluwert der Variationsspanne einer zu dieser Bergmitte benachbarten Talmitte zumindest 1,5mal so groß, um noch bessere Verschlusskraft bei hoher Flexibilität zu erzielen.

Durch eine derartige Ausgestaltung des Verhältnisses zwischen der Maximalvariation zwischen Berg und Tal einerseits und den Abständen derselben andererseits kann eine ausreichend große Variation bzw. Veränderung, wie sie vorstehend als Teil der Grundidee gefordert ist, gewährleistet werden. Dieses ermöglicht dann dementsprechend eine Maximierung der Verschlusskraft.

Gleichzeitig ist jedoch auch eine hohe Flexibilität entlang der Höhe gegeben wodurch wiederum die Verschlusskraft weiter erhöht wird. Hierbei scheinen Werte von 4 mal oder höher als nicht so sinnvoll, da damit die Berge und Täler zu viel variieren, was das Handling erschweren würde.

Auch kann sich, um Verschlussbänder bereitzustellen, welche auf baulich einfach Weise die Verschlusskraft des jeweiligen Verschlussbandes, mit welcher dieses wirken kann, maximieren, ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und sich ebenfalls parallel zur Befestigungsseite und zur Rückseite erstreckenden und senkrecht zur Breite erstreckenden und senkrecht zur Breite ausgerichteten Höhe sowie mit einer umlaufenden Verschlussbandumgrenzung, die zwei sich parallel zur Breite erstreckender Verschlussbandgrenzen und zwei sich senkrecht zur Breite erstreckender Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie die Schnittpunkte zwischen dem Flächenkomponentenrand und der Randbereichsmittenlinie trägt, variiert, wobei die Variation in Richtung der Höhe Berge und Täler aufweist und wobei der Winkel zwischen dem Flächenkomponentenrand und einer Parallelen zu der Randbereichsmittenlinie an wenigstens einem Schnittpunkt wenigstens 65° beträgt.

Auch eine derartige Ausgestaltung des Winkels zwischen Flächenkomponentenrand und Randbereichsmittenlinie kann eine ausreichend große Variation bzw. Veränderung, wie sie vorstehend als Teil der Grundidee gefordert ist, gewährleisten.

Es versteht sich, dass der Winkel vorzugsweise sogar in einem Beriech zwischen der Bergmitte und einer Talmitte über dem entsprechenden Wert liegt, was dementsprechend eine entsprechende Steilheit der Kurve in diesem Bereich bedeutet, wodurch die Variation an dieser Stelle entsprechend hoch ausgebildet sein kann.

In diesem Zusammenhang sei erläutert, dass sich der Winkel zwischen der Randbereichsmittenlinie bzw. einer Parallelen hierzu einerseits und dem Flächenkomponentenrand andererseits jeweils als der kleinsten Winkel zwischen diesen beiden Kurven in einem Schnittpunkt darstellt. Er kann also maximal 90° betragen.

Auch ist es von Vorteil, wenn entsprechende Winkel bzw. Bereiche mit entsprechenden Winkeln zwischen wenigstens zwei, insbesondere zwischen sämtlichen, Bergmitten und Talmitten liegen können.

Insbesondere kann der Winkel zwischen dem Flächenkomponentenrand und der Randbereichsmittenlinie an wenigstens einem Schnittpunkt der Randbereichsmittenlinie mit dem Flächenkomponentenrand wenigstens 65° betragen. In der Regel wird sich gerade an dem Schnittpunkt der steilste Verlauf des Flächenkomponentenrand finden, insbesondere wenn dieser harmonisch verlaufen soll. Dementsprechend kann dann durch einen entsprechend steilen Winkel auch eine entsprechend hohe Variation gewährleistet werden.

Für eine ausreichend steile Ausgestaltung der Durchgänge, kann der Winkel zwischen dem Flächenkomponentenrand und der Randbereichsmittenlinie an allen Schnittpunkten wenigstens 65° betragen.

Es versteht sich, dass mit höherem Winkel die Effekte in Bezug auf die Verschlusskraft bei hoher Flexibilität steigen, jedoch auch die Fertigung erschwert wird, sodass hier Vor- und Nachteile gegeneinander abgewogen werden müssen, um einen geeigneten Winkel zu wählen. Beispielsweise erscheint ein 90°-Winkel als besonders kompliziert bzw. sogar als maximal sinnvoller Winkel, da die Fertigung eines 90°-Winkels, wenn überhaupt realisierbar, als äußerst kompliziert erscheint.

Besonders vorteilhaft ist, wenn der Winkel wenigstens 70° beträgt, denn je höher der Winkel ist, desto größer ist die Erstreckung der ersten Flächenkomponente und somit auch die mögliche Verschlusskraft, bei hohe Flexibilität entlang der Höhe, was dementsprechend die Verschlusskraft weiter erhöht. Vorzugsweise ist der Winkel wenigstens 75°, für eine besonders vorteilhafte Ausgestaltung.

Darüber hinaus kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei mit der Randbereichsmittenlinie als Abszisse und senkrecht hierzu gerichteten Variationsspannen als Ordinate ein Koordinatensystem aufgespannt ist, in welchem der variierende Randbereich und die Randbereichsmittenlinie mit seinen Bergen und Tälern variiert und der variierende Randbereich wenigstens einen Übergang zwischen einem Berg und einem Tal aufweist, welcher zwischen 80 % der Variationsspanne der Bergmitte dieses Berges und 80 % der Variationsspanne der Talmitte dieses Tals definiert ist und welcher sich entlang der Abszisse über maximal 40 % der Summe aus diesen beiden Variationsspannen und/oder über den doppelten Mittelwert aller Variationsspannen aller Tal- und Bergmitten erstreckt, um gattungsgemäße Verschlussbänder bereitzustellen, welche auf baulich einfache Weise die Verschlusskraft des jeweiligen Verschlussbands, mit welcher dieses wirken kann, maximieren.

Im vorliegenden Zusammenhang kann unter dem "Koordinatensystem" ein rechtshändiges kartesisches Koordinatensystem verstanden werden, wobei die beiden Richtungsachsen orthogonal aufeinander stehen, sich also im 90°-Winkel schneiden. Die Koordinatenlinien sind Geraden in konstantem Abstand voneinander. Hierbei kann von der mathematischen Rechtshändigkeit ausgegangen werden, sodass vorzugsweise die horizontale Achse als Abszisse oder Abszissenachse und die vertikale Achse als Ordinate oder Ordinatenachse bezeichnet werden kann. Außerdem kann die Abszisse als x-Achse und die Ordinatenachse entsprechend als y-Achse bezeichnet werden.

Hierbei zeigt sich abermals die Umsetzung der eingangs erläuterten Grundidee, eine möglichst hohe Variation bereitzustellen, dadurch, dass insbesondere an den Flanken eines Berges bzw. Tals ein besonders steiler Bereich, also ein besonders variierenden Bereich bereitgestellt wird, der in besonderem Maße zu einer Maximierung der Verschlusskraft beiträgt.

Kumulativ bzw. alternativ zu den übrigen vorliegend genannten vorteilhaften Merkmalskombinationen kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Bereite erstreckende Verschlussbandgrenzen umfasst, um gattungsgemäße Verschlussbänder bereitzustellen, welche auf baulich einfache Weise die Verschlusskraft des jeweiligen Verschlussbands, mit welcher dieses wirken kann, maximieren, auch dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Bereite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei mit der Randbereichsmittenlinie als Abszisse und senkrecht hierzu gerichteten Variationsspannen als Ordinate ein Koordinatensystem aufgespannt ist, in welchem der variierende Randbereich um die Randbereichsmittenlinie mit seinen Bergen und Tälern variiert und die Variationsspanne wenigstens einer Bergmitte zumindest das 0,37-Fache des parallel zur Abszisse gemessenen Abstandes der beiden zu diesem Berg benachbarten Talmitten beträgt sowie der variierende Randbereich um einen Berg herum beidseits der zugehörigen Bergmitte jeweils einen 80 %-Durchgang aufweist und die beiden 80 %-Durchgänge weniger als 20 % des Abstandes der beiden zu diesem Berg benachbarten Talmitten voneinander beabstandet sind.

Durch eine derartige Ausgestaltung der Berge bzw. Täler bzw. des Flächenkomponentenrands kann ebenfalls eine ausreichend große Variation bzw. Veränderung, wie sie vorstehend als Teil der Grundidee gefordert ist, gewährleistet werden.

Vorzugsweise befindet sich der 80 %-Durchgang bei 80 % der Variationsspanne der zugehörigen Bergmitte.

Es ist von Vorteil, wenn der Abstand der beiden 80 %-Durchgänge weniger als 18 % des Abstandes der beiden zu diesem Berg benachbarten Talmitten beträgt. Hierdurch wird der Berg noch steiler bzw. spitzer was seine Wirkung im Bezug guter Verschlusskraft bei hoher Flexibilität verstärkt. Außerdem wird hierdurch eine bessere Haftung des Berges an einer zweiten Komponente eines mechanischen Verschlusssystems deutlich verstärkt, da sich herausgestellt hat, dass sich spitzere Zuläufe der Berge wesentlich besser mit der zweiten Komponente eines zweikomponentigen Verschlusssystems verbinden und dadurch die Verschlusskraft erhöht wird. In Bezug auf die spitzen Zuläufe bzw. in Bezug auf möglichst steile Flanken erscheint dieses ganz allgemein zu gelten.

Um dieselben Vorteile zu erzielen, ist insbesondere vorteilhaft, wenn der Abstand der beiden 80 %-Durchgänge weniger als 15 % des Abstandes der beiden zu diesem Berg benachbarten Talmitten beträgt.

Um gattungsgemäße Verschlussbänder bereitzustellen, welche auf baulich einfache Weise die Verschlusskraft des jeweiligen Verschlussbandes, mit welcher dieses wirken kann, maximieren, kann sich ein Verschlussband mit einer Befestigungsseite, an welcher eine erste Flächenkomponente eines zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, auch dadurch auszeichnen, dass die erste Flächenkomponente einen Flächenkomponentenrand aufweist, der wenigstens einen in der Höhe verlaufenden variierenden Randbereich aufweist, der in seinem Verlauf entlang der Höhe in Richtung der Breite um eine Randbereichsmittenlinie, die Schnittpunkte zwischen dem Flächenkomponentenrand und der Randbereichsmittenlinie trägt, variiert, wobei die Variation in Richtung der Höhe Berge und Täler aufweist und wobei wenigstens einer der Berge von seiner Bergmitte ausgehend einen losen Bereich aufweist.

Insoweit ermöglicht auch ein derartiger loser Bereich eine Veränderung derartiger Verschlussbänder gegenüber dem Stand der Technik im Lichte der eingangs genannten Grundidee, an einer der Öffnungsrichtung abgewandten Seite eine Anpassung vorzunehmen und auf diese Weise beispielsweise noch Hakenmaterial bzw. Flächenkomponente an einer der Öffnungsrichtung abgewandten Seite bereitzustellen, wo ansonsten, ohne dieses, gerade keines mehr vorzusehen wäre, wodurch dann die Verschlusskraft weiter erhöht werden kann.

Unter einem "losen Bereich" kann in vorliegendem Zusammenhang insbesondere ein Bereich verstanden werden, welcher lediglich indirekt über den Rest des Flächenelements mit dem übrigen Verschlussband verbunden ist.

Hierbei wird davon ausgegangen, dass insbesondere gegen Scherkräfte durch diese ergänzend in dem losen Bereich vorzusehende Flächenkomponente ergänzende Verschlusskräfte bereitgestellt werden können.

Es ist von Vorteil, wenn der lose Bereich von der Bergmitte ausgehend bis zur Hälfte der Variationsspanne dieser Bergmitte reicht, da diese Ausgestaltung zum einen einen besonders einfachen Aufbau ermöglicht. Zum anderen kann auf diese Weise eine ausreichend große Beweglichkeit des Bergs gewährleistet werden, der dann besonders gut zur Verstärkung der Verschlusskraft beitragen kann, indem er sich in das Gegenstück, wenn eine Zugspannung auftritt, eingraben kann. Diese Fähigkeit ist auch bei weniger stark ausgebildeten losen Bereich entsprechend vorteilhaft.

Vorzugsweise kann der lose Bereich von der Bergmitte ausgehend bis zur Hälfte der Variationsspanne dieser Bergmitte reichen, um die vorstehend genannten Vorteile zu erzielen. Je nach konkreten Verhältnissen kann der lose Bereich insbesondere von der Bergmitte ausgehend bis zu 40 % der Variationsspanne dieser Bergmitte reichen, um entsprechend vorteilhaft wirken zu können.

Vorzugsweise weisen sämtliche Berge einen von Ihrer Bergmitte ausgehenden losen Bereich auf. Dementsprechend können alle Berge auf die Verschlusskraft verstärkend wirken.

Es ist vorteilhaft, wenn die mittlere Flächenüberdeckung der Täler weniger als 50 *%* von der mittleren Flächenüberdeckung der Berge abweicht, um eine Gleichmäßigkeit der Gesamtanordnung zu ermöglichen. Durch eine gleichmäßige Gesamtanordnung kann diese auch gleichmäßig wirksam sein, da allgemein eine gleichmäßig wirksame Verschlusskraft wünschenswert bzw. vorteilhaft ist.

Kumulativ bzw. alternativ hierzu kann, um dieselben Vorteile zu erzielen, der Mittelwert aller Täler überbrückende Abstände der Schnittpunkte und der Mittelwert aller Berge überbrückende Abstände weniger als 50 % voneinander abweichen.

Es ist denkbar, dass je nach konkreter Umsetzung ggf. durch die vorstehend beschriebenen Maßnahmen insbesondere eine doppelnutzige Herstellung möglich ist.

Es ist von Vorteil, wenn die Berge und Täler zueinander symmetrisch ausgebildet sind, da hierdurch insbesondere eine doppelnutzige Herstellung ermöglicht werden kann, indem beispielsweise ein entsprechend geformter Schnitt durch das Halbzeugbandmaterial gelegt werden kann und beidseits des Schnitts ganz ähnliche Schnittmuster vorliegen.

Unter der symmetrischen Ausbildung von Bergen und Tälern kann im vorliegenden Zusammenhang beispielsweise verstanden werden, dass die Abstände von einer Bergmitte zu einer benachbarten Bergmitte gleich dem Abstand von einer Talmitte zu einer benachbarten Talmitte ist. Vorzugsweise ist auch die Variationsspanne zu einer Bergmitte gleich der Variationsspanne zu einer Talmitte.

Vorzugsweise ist in den Tälern eine zusammenhängende oder nicht zusammenhängende Klebefläche vorgesehen. Die Klebefläche kann sehr frei variiert werden. Wenn sie über die Täler an der der mechanischen Flächenkomponente abgewandten Seite hinausragen sollte, so wird sie eher durchgehend ausgebildet sein. Ist sie lediglich zwischen den Tälern angeordnet, so ist sie durch die Flächenkomponente unterbrochen. Die Erstreckung der Klebefläche parallel zur Breite kann einfach bei der Herstellung variiert werden, sodass auch die Verschlusskraft der Klebefläche entsprechend einfach an individuelle Bedürfnisse angepasst werden kann.

Unter einer "zusammenhängenden" oder "nicht zusammenhängenden Klebefläche" kann im vorliegenden Zusammenhang ein entsprechendes zusammenhängendes oder nicht zusammenhängendes Gebiet verstanden werden. Eine offene und zusammenhängende, und nicht leere, Teilmenge heißt Gebiet.

"Zusammenhängend" bedeutet dabei, dass zu je zwei beliebigen Punkten eine Kurve existiert, welche diese verbindet und "offen", dass es keine Randpunkte gibt. In einem einfach zusammenhängenden Gebiet lässt sich jede geschlossene Kurve stetig auf einen Punkt auf ihr "zusammenziehen", oder noch salopper formuliert, es darf es keine "durchgehende Löcher" oder "Henkel" geben. Eine geschlossene Kurve beispielsweise würde sich dort herum nicht zu einem Punkt zusammenziehen lassen. Entsprechend kann vorliegend die Klebefläche als Gebiet angesehen werden und die vorstehend beschriebene Bedingung geprüft werden, ob entsprechend ein zusammenhängendes Gebiet, also eine zusammenhängende Klebefläche vorliegt.

Insbesondere sei betont, dass eine durch die mechanische Flächenkomponente unterbrochene Klebefläche nicht ausschließt, dass die zugehörige Klebschicht, welche die Klebefläche bereitstellt, auch noch unterhalb der mechanischen Flächenkomponente weiterlaufen und insbesondere auch selbst dann zusammenhängend ausgebildet sein kann.

Es ist von Vorteil, wenn die Klebefläche bis in eine oder alle der jeweiligen Talmitten bzw. in die jeweilige Talmitte reicht, sodass kein klebstofffreier Bereich zwischen der Klebefläche und der Flächenkomponente vorliegt. Eine derartige Ausgestaltung ist besonders einfach zu fertigen.

Vorzugsweise ist parallel zur Breite eine Öffnungsvorzugsrichtung definiert und die Berge erstrecken sich in Öffnungsvorzugsrichtung. Die Definition der Öffnungsvorzugsrichtung kann jedoch auch schon durch die Einbaulagen oder ähnliche Randbedingungen vorgegeben sein. Die vorstehend genannte Erstreckung der Berge in Öffnungsvorzugsrichtung ermöglicht gute Verschlusskräfte auch in den Bereichen, die der Öffnungsvorzugsrichtung entgegengerichtet sind, sodass bei minimalen Materialeinsatz möglichst hohe Verschlusskräfte aufgebracht werden können.

Besonders vorteilhaft ist es, wenn die Berge über die Höhe des Verschlussbands verteilt sind, um möglichst hohe Verschlusskräfte zu erzielen.

Unter der "Öffnungsvorzugsrichtung" kann im vorliegenden Zusammenhang die Richtung verstanden werden, in der das Verschlussband vorzugsweise geöffnet wird. Es ist denkbar, dass eine Öffnungsrichtung besonders bevorzugt wird, weil ein Öffnen des Verschlussbandes in genau diese Richtung beispielsweise ein zerstörungsfreies Öffnen begünstigt. Hierbei kann die Ausgestaltung des Verschlussbandes geradeso gewählt sein, dass in der Öffnungsvorzugsrichtung weder das Verschlussband noch der Gegenstand, an dem das Verschlussband verschlossen wird, beschädigt bzw. zerstört werden. Die Öffnungsvorzugsrichtung kann hier im direkten Zusammenhang mit der restlichen Ausgestaltung des Verschlussbandes stehen, da zwar in Öffnungsvorzugsrichtung eine zerstörungsfreies Öffnen des Verschlussbandes ermöglich wird, gleichzeitig aber im verschlossenen Zustand eine gute Verschlusskraft bei hoher Flexibilität bereitstellen kann.

Insbesondere kann die Öffnungsvorzugsrichtung auch durch andere Maßnahmen, wie beispielsweise durch einen an entsprechender Stelle vorgesehenen Fingerlift oder durch eine Anordnung von Permanentbereich und Nutzerbereich nebeneinander, vorgegeben sein.

Um gattungsgemäße Verschlussbänder bereitzustellen, welche auf baulich einfach Weise die Verschlusskraft des jeweiligen Verschlussbands, mit welcher dieses wirken kann, maximieren, kann sich ein Verschlussband mit einer Befestigungsseite, an welcher eine erste Flächenkomponente einen zweikomponentigen mechanischen Flächenverschlusssystems befindlich ist, mit einer der Befestigungsseite abgewandten Rückseite und mit einer sich parallel zur Befestigungsseite und zur Rückseite erstreckenden Breite und einer sich ebenfalls parallel zur Befestigungsseite und zur Rückseite erstreckenden und senkrecht zur Breite ausgerichteten Höhe sowie mit einer umlaufenden Verschlussbandumgrenzung, die zwei sich parallel zur Breite erstreckende Verschlussbandgrenzen und zwei sich senkrecht zur Breite erstreckende Verschlussbandgrenzen umfasst, auch kumulativ bzw. alternativ zu den übrigen hier als vorteilhaft dargestellten Merkmalskombinationen dadurch auszeichnen, dass parallel zur Breite eine Öffnungsvorzugsrichtung definiert ist und an der Befestigungsseite ergänzend zu der ersten Flächenkomponente des mechanischen Flächenverschlusssystems eine zusammenhängende oder nicht zusammenhängende Klebefläche vorgesehen ist und dass von jedem Flächenpunkt der Klebefläche in Richtung der Öffnungsvorzugsrichtung kein Flächenpunkt der ersten Flächenkomponente angeordnet ist.

Hierdurch kann zum einen eine besonders gute Verschlusskraft des Verschlussbandes bei gleichzeitig hoher Flexibilität bereitgestellt werden. Zum anderen ist hierdurch gewährleistet, dass, falls bei Öffnen des Verschlussbands, die mechanische Flächenkomponente Bestandteile ihres Gegenstücks, also der zweiten Komponente, mitreißt, diese Bestandteile nur mit einer geringeren Gefahr die Klebefläche kontaminieren können. Letzteres gewahrt die Möglichkeit, dass dann das Verschlussband auch bei einem zweiten Schließvorgang wieder eine maximale Verschlusskraft bereitstellen kann.

Dieser Vorteil ergibt sich auch, falls das Verschlussband in irgendeiner Weise in einer Bereitstellungsposition vorgehalten und dort mit einer zweiten Komponente des mechanischen Flächenverschlusssystems in Kontakt ist. Wird das Verschlussband dann aus der Bereitstellungsposition gebracht, so ist ebenfalls die Gefahr eine Kontamination minimiert, wenn dieses dann in seine Verschlussposition gebracht wird.

Es ist von Vorteil, dass sich die Klebefläche in Richtung der Öffnungsvorzugsrichtung unmittelbar an die Flächenkomponente anschließt, da somit die Klebefläche dann gut als Pop-off-Sicherung für den mechanischen Verschluss dienen kann.

Unter dem "Anschließen" kann im vorliegenden Zusammenhang verstanden werden, dass zwischen der Flächenkomponente und der Klebefläche kein freier Bereich vorliegt, sondern die Klebefläche direkt in den mechanischen Verschluss übergeht.

Naturgemäß weißt die Flächenkomponente einen Flächenkomponentenrand mit einem in Öffnungsvorzugsrichtung weisenden Randbereich auf. Um einen Anordnung von der Klebefläche und dem Flächenverschluss zumindest auf gleicher Höhe entlang der Breite zu ermöglichen, ist der Randbereich vorzugsweise gekrümmt oder senkrecht zur Öffnungsvorzugsrichtung geneigt. Kumulativ bzw. alternativ hierzu kann der Randbereich variieren, um eine Anordnung von Klebefläche und Flächenverschluss zumindest auf gleicher Höhe entlang der Breite zu ermöglichen. Durch die Variation kann beispielsweise zwischen den Bereichen des Flächenverschlusses eine Klebefläche angeordnet werden, sodass sowohl Klebefläche als auch Flächenverschluss auf gleicher Höhe entlang der Breite angeordnet werden können. Es versteht sich, dass je nach Höhe unterschiedliche Verhältnisse von Klebefläche zum Flächenverschluss vorliegen können und insbesondere sogar durch den variierenden Verlauf des Randbereichs vorteilhafterweise vorliegen sollten. Es versteht sich, dass die Variation entsprechend den vorstehend erläuterten Merkmalen mit Bergen und Tälern ausgestattet sein kann, um auf diese Weise die Vorteile zu maximieren.

Vorzugsweise ist die Variation periodisch ausgebildet, da eine solche Variation besonders einfach, beispielsweise über ein Abrollen des Werkzeugs, herstellbar ist. Da ein Verschlussband selten eine Einzelstückfertigung, sondern in der Regel ein Massenprodukt ist, spielt die besonders einfache und schnelle Herstellung eine besonders wichtige Rolle.

Besonders vorteilhaft ist eine wellen- und/oder sinusförmige Variation. Eine derartig ausgebildete Variation ist in sich stabil und zudem leicht darzustellen.

Insbesondere kann die Variation Berge und Täler aufweisen, wie bereits vorstehend erläutert wurde.

Zum Verständnis der "periodischen Variation" kann im vorliegenden Zusammenhang eine Definition aus der Mathematik herangezogen werden. In der Mathematik sind periodische Funktionen eine besondere Klasse von Funktionen. Sie haben die Eigenschaft, dass sich Ihre Funktionswerte in regelmäßigen Abständen wiederholen. Die Abstände zwischen dem Auftreten der gleichen Funktionswerte werden Periode genannt. Einfache Beispiele sind Sinus- und Kosinusfunktionen.

Im vorliegenden Zusammenhang kann also unter einer "periodischen Variation" verstanden werden, dass sich Berge und Täler, insbesondere die vorstehend erläuterten Berge und Täler, in regelmäßigen Abständen wiederholen. Es versteht sich, dass auch sämtliche andere periodische Variationen theoretisch möglich sind. Beispielsweise können periodische Variationen der Flächenkomponente periodischen Signalen aus der Elektrotechnik ähneln, wie beispielsweise einer Rechteckspannung, einen Rechteckimpuls, einem Nadelimpuls, einer Dreiecksspannung, einer Sägezahnspannung, einer Trapezspannung, einer Treppenspannung, einer Einweggleichrichtung sowie einer Zweiwegegleichrichtung von Sinusspannung. Die entsprechenden periodischen Signale aus der Elektrotechnik können als geometrisches Vorbild zur Ausgestaltung der Variation der Flächenkomponente dienen, da jede dieser Verläufe einen periodischen Verlauf aufweist. Es versteht sich jedoch, dass je nach Verlauf die Herstellung desselben besonders einfach, schwieriger oder praktisch kaum umsetzbar herzustellen ist. So sind beispielsweise Rechteckspannungen bzw. Verläufe rein praktisch nur sehr schwer herzustellen.

Wenn die Variation der Flächenkomponente als wellen- und/oder sinusförmig aufgefasst wird, kann eine derartige Wellenform der Variation auch über Größen beschrieben werden, die zur mathematischen Beschreibung von Wellen nötig sind, wie beispielsweise Amplitude, Phase, Wellenlänge bzw. Ausbreitungs- oder Phasengeschwindigkeit.

Vorteilhafterweise ist zwischen einem Tal bzw. einer zugehörigen Talmitte und der Klebefläche ein klebstofffreier Bereich angeordnet. Auf diese Weise bildet die Klebefläche einen Streifen, der sowohl auf seiner Seite in Öffnungsvorzugsrichtung als auch auf der der Öffnungsvorzugsrichtung gegenüberliegenden Seite einen klebstofffreien Bereich aufweist und somit dort nicht unmittelbar an einen weiteren Bereich, wie beispielsweise an der Flächenkomponente anschließt. Eine solche als Streifen ausgebildete Klebefläche hat sich als besonders vorteilhaft darin erwiesen, eine hohe Verschlusskraft bereitzustellen, ohne dabei beim Öffnen des Verschlussbandes dieses bzw. die Komponente, an welcher das Verschlussband angeklebt wird, zu beschädigen.

Hierbei wird die Klebefläche in der Regel durch eine Klebstoffschicht bereitgestellt, die ggf. auch der Befestigung der Flächenkomponente dienen kann. Bei einer derartigen Ausgestaltung kann dann insbesondere auch unterhalb der Flächenkomponente keine Befestigung bzw. kein Klebstoff vorgesehen sein, so dass diese dort an sich frei beweglich verbleibt, während im Bereich des Streifens dann auch die Klebstofffläche, je nach konkreter Ausgestaltung, bis unter die Flächenkomponente reichen und letztere befestigen kann. Es hat sich herausgestellt, dass, bei einer geeigneten Dimensionierung des Klebestreifens einerseits eine gute Befestigung der Flächenkomponente und andererseits eine gute Beweglichkeit derselben gewährleistet werden kann, wobei letzteres hinsichtlich der Halteeigenschaften der Flächenkomponente vorteilhaft ist.

Es versteht sich, dass diese als Streifen ausgebildete Klebefläche in ihrer Position veränderbar ist. So kann die Klebefläche in Öffnungsvorzugsrichtung auf unterschiedlicher Höhe angeordnet sein. Aus den unterschiedlichen Anordnungen können sich jeweils andere Vorteile bezüglich der Verschlusskraft entsprechend der Anwendung ergeben.

Vorzugsweise erstreckt sich die Klebefläche in Öffnungsvorzugsrichtung bis zu den Bergen. Es ist jedoch auch denkbar, dass sich diese in Öffnungsvorzugsrichtung über die Berge hinaus bzw. bis vor die Berge erstreckt. Hieraus können sich unterschiedliche Vorteile bei der Ausgestaltung der Verschlusskraft ergeben.

Es ist vorteilhaft, wenn sich die Flächenkomponente in Richtung der Öffnungsvorzugsrichtung weiter erstreckt als die Klebefläche. Dieses ermöglicht eine gute Verschlussfähigkeit des mechanischen Verschlusses. Gegebenenfalls ist dann unter der Flächenkomponente noch ergänzend Klebstoff vorgesehen, damit diese ausreichend befestigt ist. Es ist jedoch auch denkbar, dass der Bereich der Flächenkomponente, der sich in Richtung der Öffnungsvorzugsrichtung weitererstreckt, als die Klebefläche gerade nicht mit ergänzendem Klebstoff versehen ist, sodass dieser Bereich lose ist. Ein derartig loser Bereich kann gegebenenfalls die Verschlusskraft sogar erhöhen, da sich die losen Enden und insbesondere die schmal zulaufenden Endbereiche der Flächenkomponente besser in einer zweiten Komponente des mechanischen Verschlusssystems verhaken bzw. eingraben können.

Vorzugsweise erstreckt sich der lose Bereich von der Bergmitte ausgehend bis zum Beginn der Klebfläche. Dieses ermöglicht einen besonders einfachen Aufbau, da dann eine Klebstoffschicht doppelt genutzt werden kann, einerseits, um die Klebefläche bereitzustellen, andererseits, um die Flächenkomponente zu befestigen.

Gleichzeitig kann der lose Bereich entsprechend die Verschlusskraft fördernd eingesetzt werden, da sich insbesondere die schmal zulaufenden Enden des losen Bereiches verstärkt in die zweite Komponente des mechanischen Verschlusssystems bzw. in das Objekt, auf dass das Verschlussband aufgebracht wird, eingraben. Hierdurch wird die Verschlusskraft deutlich erhöht. Dementsprechend folgt auch, dass es von Vorteil ist, wenn die losen Bereiche möglichst mit steilen Bergflanken versehen und/oder möglichst schmal sind, um ein gutes Eingraben zu ermöglichen.

Auch kann die Öffnungsvorzugsrichtung durch das Anordnen eines Anfassers an dem Verschlussband definiert sein. Dieser Anfasser kann ergriffen werden, wobei dann durch Ziehen oder Betätigen des Anfassers der Flächenverschluss geöffnet werden kann. Es versteht sich, dass das Verschlussband auch theoretisch ohne Anfasser öffenbar ist, das Öffnen jedoch durch den Anfasser erheblich vereinfacht wird. Durch eine entsprechende Anordnung des Anfassers ist die bevorzugte Öffnungsrichtung direkt definiert, da naturgemäß ein Verschlussband abgezogen werden muss und durch die Anordnung des Anfassers an einem Ende des Verschlussbandes die Öffnungsvorzugsrichtung unmittelbar vorgegeben ist. Ein Öffnen entgegen dieser Richtung bei einem entsprechend angeordneten Verschlussband, ist möglicherweise zwar nicht ausgeschlossen, da Personen sicherlich auch gegen Empfehlungen und mechanische Vorgaben verstoßen können. Anderenfalls ist es dann natürlich nicht mehr zwingend, dass erfindungsgemäße Vorteile entsprechend genutzt werden können.

Es ist von Vorteil, wenn der Anfasser an der entgegen der Öffnungsvorzugsrichtung angeordneten Verschlussbandgrenze angeordnet ist, da durch Ziehen des Anfassers der Flächenverschluss bzw. die anhaftenden Klebefläche von dem Anfasser ausgehend in gewünschter Weise abgehoben werden können und ein entsprechender Verschluss geöffnet werden kann.

Ein Anfasser kann als Fingerlift ausgestaltet sein, welcher bereits aus dem Stand der Technik bekannt ist, jedoch eine bewährte Ausgestaltung eines Anfassers darstellt. Andererseits versteht es sich, dass ein Anfasser auch durch einen entsprechend ausgestalteten Knopf oder durch eine entsprechend ausgestaltete Schlinge oder durch ähnliche Maßnahmen bereitgestellt werden kann.

Besonders bevorzugt kann als Fingerlift ein an- oder aufgesetztes Griffband eingesetzt werden. Auch eine gewellte Verschlussbandgrenze kann der Fingerlift umfassen bzw. als Fingerlift dienen, da sich eine solche ebenfalls als bewährte Ausgestaltung eines Anfassers herausgestellt hat.

Es versteht sich, dass die Ausgestaltung des Anfassers den entsprechenden Einsatzgebieten bzw. Anwendungen angepasst werden kann und entsprechend beliebig ausgestaltet werden können, wobei es naturgemäß von Vorteil ist, wenn der Anfasser für die anfassende Person leicht anzufassen bzw. zu greifen ist.

Vorzugsweise ist die erste Flächenkomponente ein Hakenmaterial mit von einer Trägerbahn getragenen Haken oder ein Schlaufenmaterial, da Haken und Schlaufen Bestandteile eines kostengünstigen und dennoch zuverlässigen zweikomponentigen Flächenverschlusssystems aus Haken und Schlaufen darstellen. Dementsprechend kann die zweite Komponente ein entsprechendes Schlaufenmaterial bzw. Hakenmaterial sein. Je nach konkreter Ausgestaltung können diesbezüglich alle möglichen aus dem Stand der Technik bekannten Kombination vorteilhaft zum Einsatz kommen, wie beispielsweise zwei kongruente Hakenmaterialien als die beiden Komponenten oder ein ausreichend Hinterschneidungen aufweisendes NonWoven als eine der Komponenten. Im Bereich der Verschlussbänder haben sich die Schlaufen-Haken-Verbindungen als mechanisches zweikomponentiges Flächenverschlusssystem bewährt und neben Klebstoffschichten als bevorzugte Ausgestaltung durchgesetzt.

Zudem kann die erste Flächenkomponente als zusammenhängendes Gebiet ausgebildet sein. Hieraus folgen ein umlaufender Flächenkomponentenrand und eine vorteilhafte kompakte Ausgestaltung der ersten Flächenkomponente.

In einer besonders vorteilhaften Ausführungsform kann die erste Flächenkomponente als einfach zusammenhängendes Gebiet ausgebildet sein. Hieraus folgen keine singulären Löcher oder Inseln, sodass die Flächenkomponente insbesondere vor Ihrer Applikation an einem Trägerband einfach gehandhabt werden und außerdem der Verschnitt minimiert werden kann.

Für eine einfache Bereitstellung und Applikation des Verschlussbandes kann das Verschlussband zudem ein Trägerband umfassen, welches die erste Flächenkomponente sowie gegebenenfalls eine die Klebefläche bildende Klebstoffschicht trägt.

Kumulativ bzw. alternativ hierzu kann das Trägerband auch den Anfasser tragen, wodurch insgesamt eine einfache Bereitstellung und Applikation des Verschlussbandes erzielt werden kann, da über das Trägerband sämtliche Komponenten des Verschlussbandes entsprechend einfach bereitgestellt werden. Wird der Anfasser lediglich durch einen entsprechend ausgebildeten Verschlussbandrand bereitgestellt, kann dieser Verschlussbandrand beispielsweise durch das Trägerband bereitgestellt werden.

Es ist vorteilhaft, wenn das Verschlussband ein Permanentbereich zur permanenten Befestigung an einem mit dem Verschlussband zu verschließenden Gegenstand, wie einem Kleidungsstück oder einem Hygieneartikel, und ein Benutzerbereich zur lösbaren Befestigung an dem Gegenstand aufweist. Auf diese Weise kann das Verschlussband permanent an einem entsprechenden Gegenstand befestigt und über den Benutzerbereich nach Bedarf geöffnet und geschlossen werden.

Hierbei können der Permanentbereich und der Benutzerbereich senkrecht zur Befestigungs- und zur Rückseite überschneidend oder voneinander beabstandet angeordnet sein. Die überschneidende Anordnung ermöglicht einen sehr kompakten und stabilen Verschluss, wobei die permanente Befestigung an dem Gegenstand dann an der Rückseite erfolgt. Durch die voneinander beabstandete Anordnung wird ein übergreifender Verschluss ermöglicht, wobei die permanente Befestigung an dem Gegenstand je nach Präferenz auf der Rückseite und/oder Befestigungsseite oder sogar an beiden Seiten erfolgen kann.

Um gattungsgemäße Verschlussbänder bereitzustellen, welche auf baulich einfache Weise die Verschlusskraft des jeweiligen Verschlussbandes, mit welcher dieses wirken kann, maximieren, kann bei einem Halbzeugbandmaterial mit einer Maschinenrichtung und einer senkrecht hierzu angeordneten Querrichtung zur Herstellung eines Verschlussbandes mittels Trennen des Halbzeugbandmaterials in Querrichtung die Maschinenrichtung der Höhe und die Querrichtung der Breite des Verschlussbandes entsprechen. Das entsprechende Halbzeugbandmaterial kann leicht transportiert und gelagert werden, beispielsweise in Schlaufen, Rollen oder Spulen. Durch Trennen in Querrichtung kann dann ein entsprechendes Verschlussband hergestellt werden, ggf. nachdem durch einen Schnitt bei doppelnutziger Ausgestaltung des Halbzeugbandmaterials entlang der Maschinenrichtung erst das Halbzeugbandmaterial geteilt wurde. Insbesondere das Trennen in Querrichtung kann dann vor Ort, unmittelbar vor einer Applikation bzw. vor einem Einsatz des Verschlussbands erst erfolgen.

Auch kann ein Verfahren zur Abstimmung der Klebekräfte bei einem Verschlussband sich durch Variation der Überlappung zwischen der ersten Flächenkomponente und einer die Klebefläche bildenden unter der ersten Flächenkomponente angeordneten Klebstoffschicht auszeichnen, um gattungsgemäße Verschlussbänder bereitzustellen, welche auf bauliche einfache Weise die Verschlusskraft des jeweiligen Verschlussbandes, mit welcher diese wirken kann, maximieren. Hierbei ermöglich die einfache Abstimmung der Klebekräfte eine Anpassung an verschiedene Gegebenheiten bzw. Kundenwünsche. Dieses insbesondere deswegen einfach, da die Position bzw. Breite einer Klebestoffschicht maschinentechnisch verhältnismäßig einfach einstellbar ist, da dieses in Querrichtung erfolgt.

Es versteht sich, dass die Merkmale der vorstehend bzw. in den Ansprüchen beschriebenen Lösungen gegebenenfalls auch kombiniert werden können, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:
- Figur 1: eine schematische Darstellung eines ersten Verschlussbandes;
- Figur 2: die Darstellung nach Figur 1 nochmals, der Übersicht halber;
- Figur 3: eine weitere Darstellung des Verschlussbandes nach den Figuren 1 und 2 nochmals, der Übersicht halber;
- Figur 4: eine Darstellung des variierenden Randbereichs des ersten Verschlussbandes nach Figuren 1 bis 3 in einem Koordinatensystem;
- Figur 5: eine schematische Darstellung eines zweiten Verschlussbandes mit einem Benutzerbereich und mit einem Permanentbereich, wobei die Berge jeweils lose Bereiche aufweisen und eine nicht zusammenhängende Klebefläche vorgesehen ist;
- Figur 6: einen Querschnitt des Verschlussbandes nach Figur 5;
- Figur 7: eine schematische Darstellung eines dritten Verschlussbandes mit einem Benutzerbereich und mit einem Permanentbereich, wobei die Klebefläche bis zu den Bergmitten reicht und die Berge zur Gänze mit der Trägerbahn verbunden sind;
- Figur 8: einen Querschnitt des Verschlussbandes nach Figur 7;
- Figur 9: eine schematische Darstellung eines vierten Verschlussbandes mit einem Benutzerbereich und mit einem Permanentbereich, wobei die Klebefläche sich in Öffnungsvorzugsrichtung über die Berge hinaus erstreckt und die Berge zur Gänze mit der Trägerbahn verbunden sind;
- Figur 10: einen Querschnitt des Verschlussbandes nach Figur 9;
- Figur 11: eine schematische Darstellung eines fünften Verschlussbandes mit einem Benutzerbereich und mit einem Permanentbereich, wobei die Berge keine lose Bereiche aufweisen, aber eine nicht zusammenhängende Klebefläche vorgesehen ist;
- Figur 12: einen Querschnitt des Verschlussbandes nach Figur 11, wobei aus zeichnerischen Gründen zwischen der Trägerbahnklebstoffschicht ein Spalt dargestellt ist, der in der Realität aufgrund der Biegsamkeit, Elastizität, Kompressibilität und Stärke der Komponenten nicht in dieser Form vorhanden ist, so dass die Flächenkomponente auch dort unmittelbar mit dem Trägerband verklebt ist und allenfalls in dem Zwickel zwischen den Klebstoffschichten und der Trägerbahn eine kleine offene Stelle verbleiben kann;
- Figur 13: eine schematische Darstellung eines sechsten Verschlussbandes mit einem Benutzerbereich und mit einem Permanentbereich, wobei die Berge lose Bereiche aufweisen, eine nicht zusammenhängende Klebefläche vorgesehen ist und ein klebstofffreier Bereich zwischen Klebefläche und der Flächenkomponente angeordnet ist;
- Figur 14: einen Querschnitt des Verschlussbandes nach Figur 13;
- Figur 15: eine schematische Darstellung eines siebten Verschlussbandes mit einem Benutzerbereich und mit einem Permanentbereich, wobei die Berge keinen losen Bereich aufweisen und ein klebstofffreier Bereichen zwischen Klebefläche und der Flächenkomponente vorgesehen ist;
- Figur 16: einen Querschnitt des Verschlussbandes nach Figur 15;
- Figur 17: eine perspektivische Darstellung einer Halbzeugrolle;
- Figur 18: eine schematische Darstellung einer Halbzeugspule;
- Figur 19: eine perspektivische Darstellung einer Windel im geschlossenen Zustand;
- Figur 20: s eine chematische Darstellung einer eingelegten Damenbinde;
- Figur 21: eine schematische Darstellung einer zweiten Damenbinde mit parallelen Flügelverschlüssen in geöffnetem Zustand; und
- Figur 22: eine schematische Darstellung einer dritten Damenbinde mit versetzten Flügelverschlüssen in geöffnetem Zustand.

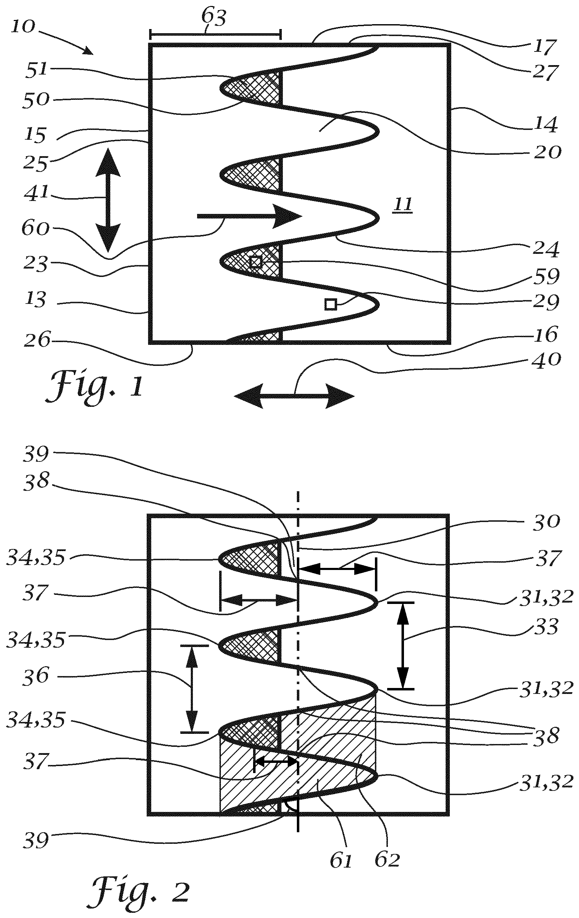

In einem ersten Ausführungsbeispiel eines Verschlussbands 10, wie es in den Figuren 1 bis 4 dargestellt ist, hat das Verschlussband 10 eine rechteckige Form und weist eine Befestigungsseite 11 sowie eine hier nicht dargestellte Rückseite 12 auf.

Das Verschlussband 10 erstreckt sich in eine Breite 40 sowie in eine Höhe 41.

Auf der Befestigungsseite 11 des Verschlussbandes 10 ist eine erste Flächenkomponente 20 eines mechanischen zwei-komponentigen Verschlusssystems, wie beispielsweise ein Haken-Material eine Haken-Schlaufen-Verschlusssystems, angeordnet.

Das Verschlussband 10 weist eine umlaufende Verschlussbandumgrenzung 13, welche sich aus vier Verschlussbandgrenzen 14, 15, 16, 17 zusammensetzt. Die Verschlussbandgrenzen 14 und 15 verlaufen parallel zueinander und parallel zur Höhe 41 so, wie auch die Verschlussbandgrenzen 16 und 17 parallel zueinander und parallel zur Breite 40 verlaufen, wobei jedoch die Verschlussbandgrenzen 14 und 15 senkrecht zu den Verschlussbandgrenzen 16 und 17 angeordnet sind, wodurch die rechteckige Grundform des Verschlussbandes 10 erzeugt wird.

Die erste Flächenkomponente 20 weist einen Flächenkomponentenrand 23 auf, der zwei parallele sich in die Breite 40 erstreckende Randbereiche 26, 27, einen senkrecht zu den beiden Randbereichen 26, 27 in die Höhe 41 erstreckenden Randbereich 25 sowie einen senkrecht zu den beiden Randbereichen 26, 27 in die Höhe 41 variierenden Randbereich 24 umfasst.

Zudem umfasst die erste Flächenkomponente 20 unendlich viele Flächenpunkte 29, wobei in der Figur 1 exemplarisch ein Flächenpunkt 29 dargestellt ist.

Darüber hinaus ist auf der Befestigungsseite 11 des Verschlussbandes 10 an der ersten Flächenkomponente 20 angrenzend eine Klebefläche 50 angeordnet, die durch eine Klebstoffschicht 51 bereitgestellt ist. Diese umfasst unendlich viele theoretische Flächenpunkte 59, wobei exemplarisch in der Figur 1 ein Flächenpunkt 59 dargestellt ist.

Von jedem Flächenpunkt 59 der Klebefläche 50 ist bei diesem Ausführungsbeispiel in Richtung einer Öffnungsvorzugsrichtung 60 kein Flächenpunkt 29 der ersten Flächenkomponente 20 angeordnet.

Der variierende Randbereich 24, der entlang der Höhe 41 verläuft, variiert um eine Randbereichsmittelinie 30, die Schnittpunkte 38 zwischen dem Flächenkomponentenrand 23 und der Randbereichsmittellinie 30 trägt.

Zudem weist die Variation in Richtung der Höhe 41 bzw. in seinem Verlauf entlang der Höhe 41 Berge 31 und Täler 34 auf.

Zudem weist die Variation des variierenden Randbereichs 24 Variationsspannen 37 auf, wie sie in den Figuren 2 bis 4 dargestellt sind. Die Variationsspannen 37 liegen zwischen Berge 31 und Täler 34 zu der Randbereichsmittellinie 30 und sind als der Abstand zwischen Randbereichsmittellinie 30 und dem variierenden Randbereich 24 bestimmt.

Jeder Berg 31 umfasst eine Bergmitte 32 so, wie jedes Tal 34 eine Talmitte 35 umfasst. Hierbei bildet das Maximum des Berge 31 die Bergmitte 32 und entsprechend das Minimum des Tals 34 die Talmitte 35. Wenn keine eindeutigen Maxima bzw. Minima zu finden sind, lassen sich die Tal- bzw. Bergmittel 32, 35 als die Mitte dieser auf einer Geraden liegenden Punkte definieren.

Die Bergmitten 32 sind jeweils in einem Abstand 33 voneinander angeordnet und die Talmitten 35 sind in einem Abstand 36 voneinander angeordnet.

Zudem beträgt ein Winkel 39 zwischen dem Flächenkomponentenrand 23 und der Randbereichsmittellinie 30 an den Schnittpunkten 38 wenigstens 65°.

Im vorliegenden ersten Ausführungsbeispiel übersteigt die Zahl der Berge 31 und die Zahl der Täler 34 2 pro 1 cm Erstreckung des Randbereichs 25.

Des Weiteren ist die Summe aus dem Absolutwert der Variationsspanne 37 einer Bergmitte 32 und dem Absolutwert der Variationsspanne 37 einer zur dieser Bergmitte 32 benachbarten Talmitte 35 größer, wie der doppelte Abstand parallel zur Höhe 41 zwischen dieser Talmitte 35 und dieser Bergmitte 32 und größer wie die mittleren Abstände der Bergmitten 32 und auch wie die mittleren Abstände der Talmitten 35.

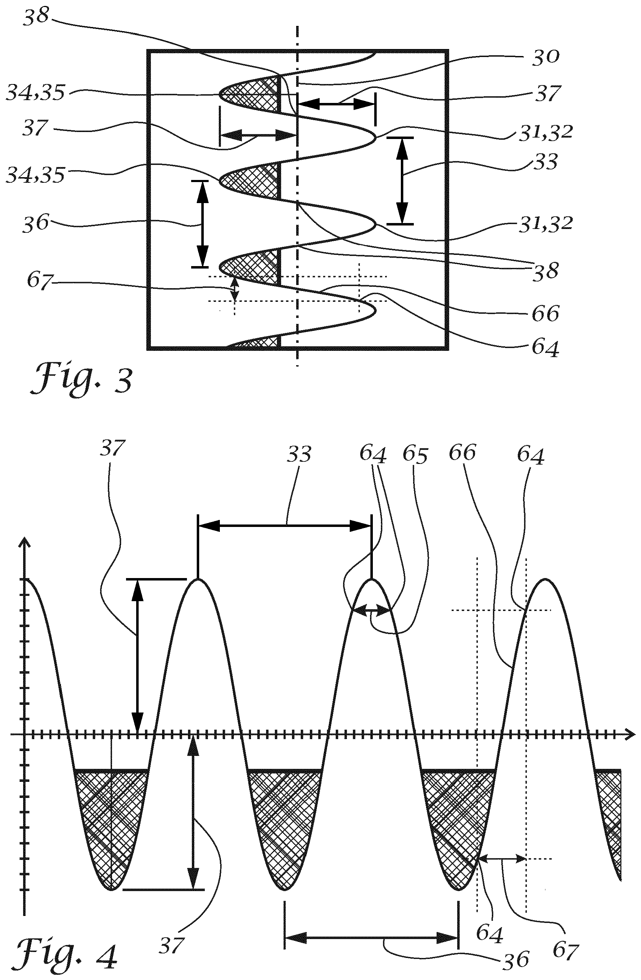

Wie in der Figur 4 des ersten Ausführungsbeispiels nach den Figuren 1 bis 4 dargestellt ist, ist mit der Randbereichsmittellinie 30 als Abszisse und senkrecht hierzu gerichteten Variationsspannen 37 als Ordinate ein Koordinatensystem aufgespannt. In diesem variiert der variierende Randbereich 24 um die Randbereichsmittellinie 30 mit seinen Bergen 31 und Tälern 34.

Der variierende Randbereich 24 weist hierbei wenigstens einen Übergang 66 zwischen einem Berg 31 und einem Tal 34 auf, welcher zwischen 80 % der Variationsspanne 37 der Bergmitte 32 dieses Berges 31 und 80 % der Variationsspanne 37 der Talmitte 35 dieses Tals 34 definiert ist. Der Übergang 66 erstreckt sich entlang der Abszisse mit einer Erstreckung 67 über maximal 40 % der Summe aus diesen beiden Variationsspannen 37 und über den doppelten Mittelwert aller Variationsspannen 37 aller Tal- und Bergmitten 32, 35.

Darüber hinaus beträgt die Variationsspanne 37 der Bergmitten 32 zumindest das 0,37 fache des parallel zur Abszisse gemessenen Abstandes 36 der beiden zu diesem Berg 31 benachbarten Talmitten 35.

Auch weist der variierende Randbereich 24 um einen Berg 31 bzw. um ein Tal 34 herum beidseits der zugehörigen Bergmitte 32 bzw. der zugehörigen Talmitte 35 jeweils einen 80%-Durchgang 64 auf. Dieser liegt bei 80 % der Variationsspanne 37 der zugehörigen Bergmitte 32 bzw. Talmitte 35.

Die beiden 80 % Durchgänge 64 sind weniger als 20 % des Abstandes 36 der beiden zu diesem Berg 31 benachbarten Talmitten 35 voneinander um einen Abstand 65 beabstandet.

Die Randbereichsmittenlinie 30 wird aus dem Mittelwert des variierenden Randbereichs 24 in Bezug auf die Höhe 41 bestimmt, wie dieses in Formel (3) dargestellt ist.

Des Weiteren lässt sich ein Mittelvariationswert entsprechend den Formeln (6) bis (8) bestimmen, der dem Mittelwert der absoluten Variationsspannen 37 über der Erstreckung des variierenden Randbereichs 24 darstellt.

In vorliegendem Ausführungsbeispiel nach den Figuren 1 bis 4 ist der Mittelvariationswert größer, als der 0,38-fache mittlere Abstand 33 parallel zur Höhe 41 zwischen zwei benachbarten Bergmitten 32 und zwischen zwei benachbarten Talmitten 35.

Die Berge 31 und Täler 34 des vorliegenden Ausführungsbeispiels sind zudem zueinander symmetrisch ausgebildet.

In den Tälern 34 ist die Klebefläche 50 als eine nicht zusammenhängende Klebfläche 50 ausgebildet.

Außerdem ist parallel zur Breite 40 eine Öffnungsvorzugsrichtung 60 definiert und die Berge 31 erstrecken sich über die Höhe 41 verteilt in Öffnungsvorzugsrichtung 60.

Von jedem Flächenpunkt 59 der Klebefläche 50 ist bei diesem Ausführungsbeispiel in Richtung dieser Öffnungsvorzugsrichtung 60 kein Flächenpunkt 29 der ersten Flächenkomponente 20 angeordnet, wie bereits vorstehend angedeutet.

Die Variation des variierenden Randbereichs 24 ist sinus- bzw. wellenförmig ausgestaltet.

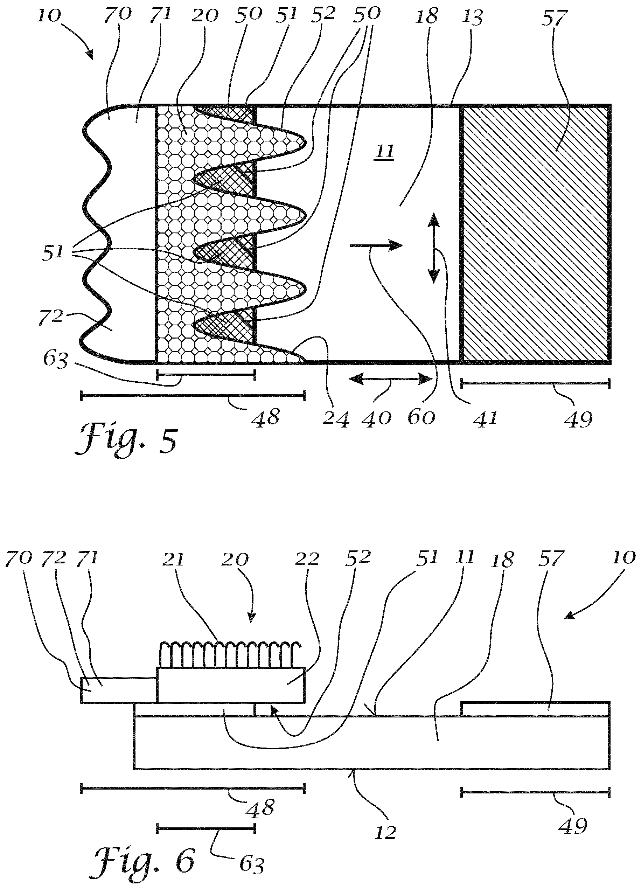

In dem zweiten Ausführungsbeispiel nach den Figuren 5 bis 6 ist ein zweites Verschlussband 10 mit einer vorgesehen Anordnung, die grundsätzlich der Anordnung des ersten Verschlussbandes nach den Figuren 1 bis 4 entspricht, so dass auf eine Wiederholung an dieser Stelle verzichtet wird.

Zusätzlich umfasst ein Trägerband 18, was auch bei dem Ausführungsbeispiel nach Figuren 1 bis 4 in dieser Form oder mit überlappenden Permanent- und Benutzerbereichen vorgesehen sein kann, neben einem Benutzerbereich 48 auch einen Permanentbereich 49, wobei in dem Permanentbereich 49 eine Klebstoffschicht 57 vorgesehen, welche eine permanente Befestigung an einem mit dem Verschlussband 10 zu verschließenden Gegenstand ermöglicht.

Außerdem erstreckt sich die Flächenkomponente 20 in Richtung der Öffnungsvorzugsrichtung 60 weiter als die Klebefläche 50 bzw. der zugehörigen Klebstoffschicht 51, welche auch die Flächenkomponente 50 an dem Trägerband 18 befestigt, sodass eine Überlappung 63 zwischen ersten Flächenkomponente 20 und Klebstoffschicht 51 vorliegt. Der hierrüber sich hinaus erstreckende Bereich der Flächenkomponente 20 bildet einen losen Bereich 52. Somit erstreckt sich der lose Bereich 52 von der Bergmitte 32 ausgehend bis zum Beginn der Klebefläche 50.

Außerdem ist im vorliegenden Ausführungsbeispiel im Vergleich zum ersten Ausführungsbeispiel nach den Figuren 1 bis 4 an der entgegen der Öffnungsvorzugsrichtung 60 angeordneten Verschlussbandgrenze 15 ein an-oder aufgesetztes Griffband 72 als Anfasser 70 und somit als Fingerlift 71 angeordnet. Durch diesen Anfasser 70 und den Permanentbereich 49 wird im Prinzip die Öffnungsvorrichtung 60 bei diesem Ausführungsbeispiel definiert.

Bei den vorliegenden Ausführungsbeispielen ist die erste Flächenkomponente 20 ein Hakenmaterial eines Haken-Schlaufen-Verschlusssystems mit von einer Trägerbahn 22 getragenen Haken 21. Es versteht sich, dass hier auch ohne weiteres andere Ausführungsformen möglich sind, solange ein Flächenverschluss gebildet werden kann. Insbesondere könnten auch Schlaufen oder andere entsprechende Verschlusseinrichtungen auf einer entsprechenden Trägerbahn vorgesehen sein.

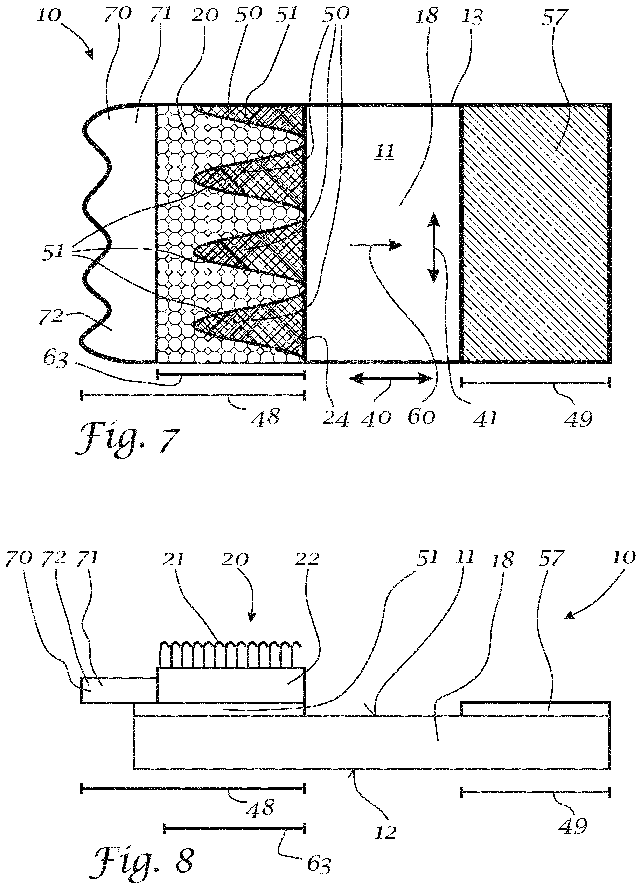

In einem dritten Ausführungsbeispiel nach den Figuren 7 und 8 ist ein grundlegend ähnliches Verschlussband 10, wie das zweite Verschlussband 10 nach dem zweiten Ausführungsbeispiel nach den Figuren 5 und 6 vorgesehen, wobei die Klebefläche 50 bis zu den Bergmitten 33 reicht und die Berge 31 zur Gänze mit der Trägerbahn 22 verbunden sind. Dementsprechend ist in dem vorliegenden Ausführungsbeispiel kein loser Bereich 52 vorhanden.

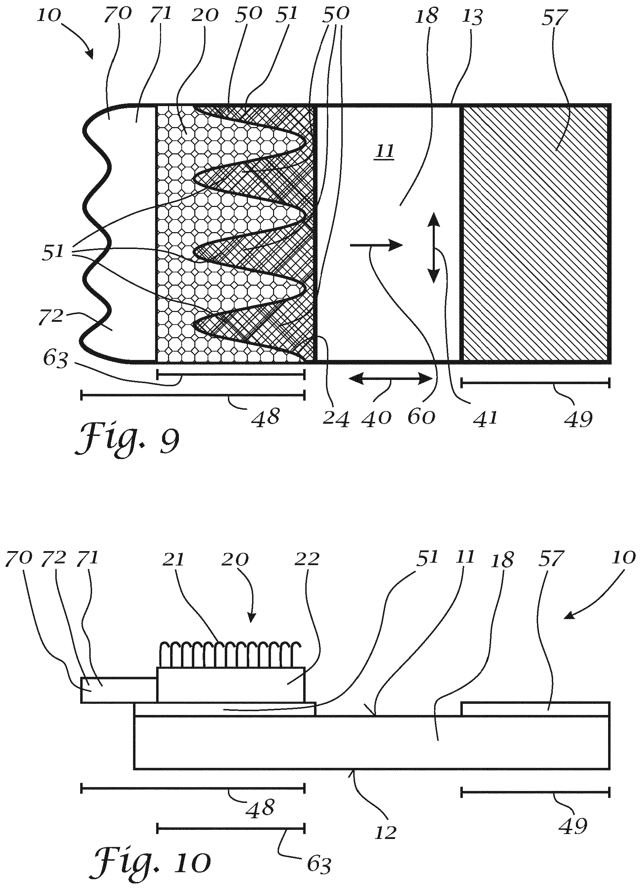

In einem vierten Ausführungsbeispiel nach den Figuren 9 und 10 ist ein weiteres Verschlussband 10 vorgesehen, welches sich zu dem dritten Verschlussband nach den Figuren 7 und 8 dadurch unterscheidet, dass die Klebefläche 50 sich in Öffnungsvorzugsrichtung 60 über die Berge 32 hinauserstreckt und die Berge 32 zur Gänze mit der Trägerbahn 22 verbunden sind. Hierdurch bildet die Klebefläche 50 ein zusammenhängendes Gebiet, also eine zusammenhängende Klebefläche 50. Es versteht sich, dass auch im vorliegenden Ausführungsbeispiel keine losen Bereiche 52 vorliegen können.

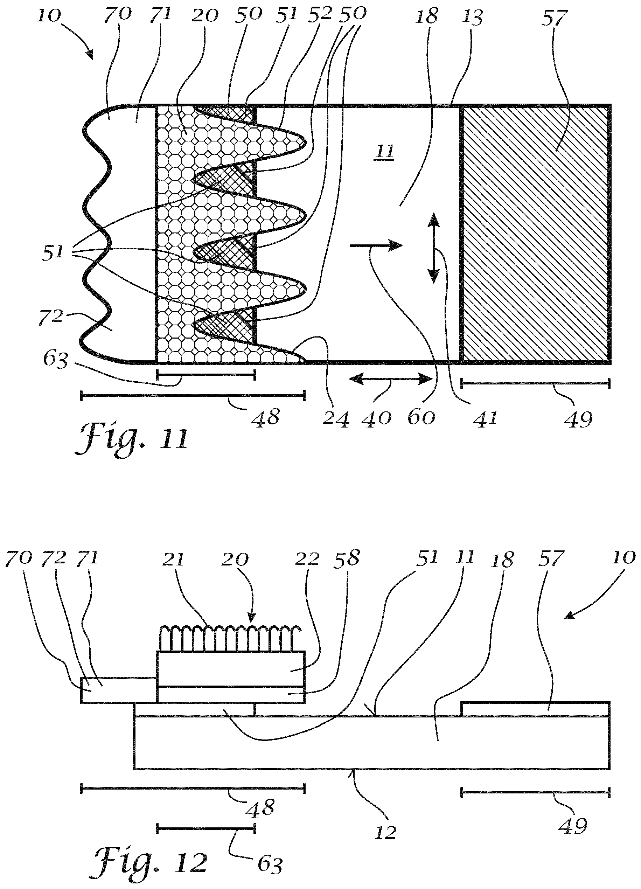

In einem weiteren Ausführungsbeispiel nach den Figuren 11 und 12 ist ein Verschlussband 10 vorgesehen, welches grundlegend dem Verschlussband nach den Figuren 5 und 6 entspricht, wobei die Berge 32 keine losen Bereiche 52 aufweisen. Auch in diesem Ausführungsbeispiel ist eine nicht zusammenhängende Klebefläche 50 vorgesehen.

In der Darstellung der Figur 11 ist aus zeichnerischen Gründen zwischen einer ergänzend vorgesehenen Trägerbahnklebstoffschicht 58, mit welcher die Trägerbahn 22 der Haken 21 an dem Trägerband 18 unmittelbar und mittelbar über die Klebstoffschicht 51 verbunden ist, und dem Trägerband 18 ein Spalt dargestellt, der in der Realität aufgrund der Biegsamkeit, Elastizität, Kompressibilität und Stärker der Komponenten nicht in dieser Form vorhanden ist, sodass die Flächenkomponente 20 auch dort unmittelbar mit dem Trägerband 18 verklebt ist und allenfalls in dem Zwickel zwischen den Klebstoffschichten 51, 58 eine kleine offene Stelle verbleiben kann.

Außerdem weicht bei allen Verschlussbändern der vorstehend genannten Ausführungsbeispiele die mittlere Flächenüberdeckung 62 der Täler 34 weniger als 50 % von der mittleren Flächenüberdeckung 61 der Berge 31 ab und der Mittelwert aller Täler 34 überbrückende Abstände der Schnittpunkte 38 und der Mittelwert aller Berge 31 überbrückende Abstände weichen weniger als 50 % voneinander ab.

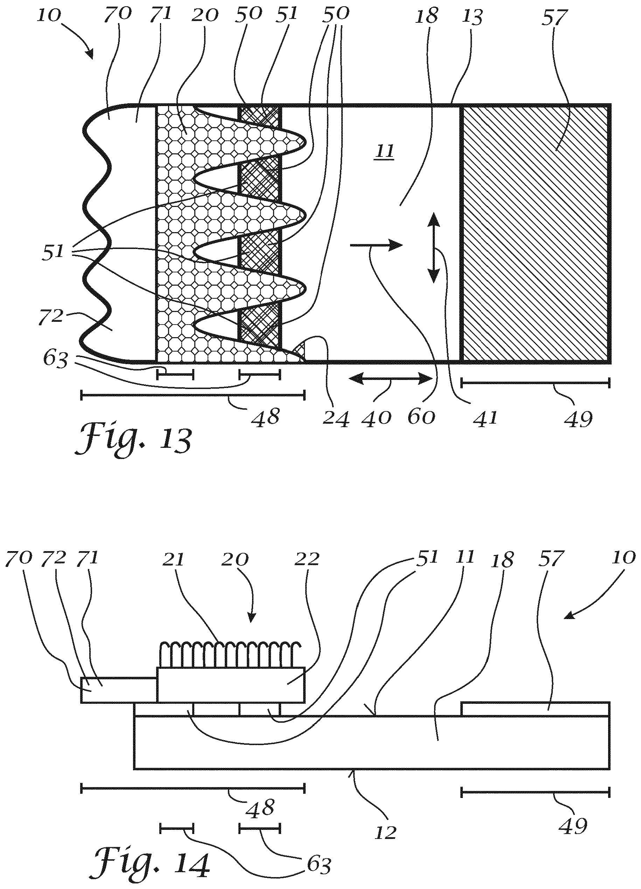

In einem weiteren Ausführungsbeispiel, welches in den Figuren 13 und 14 dargestellt ist, ist ein Verschlussband 10 vorgesehen, welches grundlegend dem Verschlussband nach den Figuren 11 und 12 entspricht, wobei zwischen der Flächenkomponente 20 und der Klebefläche 50 ein klebstofffreier Bereich zu finden ist. Eine Klebefläche 50 bildenden Klebstoffschicht 51 umfasst dann im Wesentlichen zwei Streifen, wobei einer der Streifen unter der Flächenkomponente 20 und dem Anfasser 70 angeordnet ist und der andere der beiden Streifen sowohl der Befestigung der Berge 31 als auch der Bildung der Klebefläche 50 dient.

Somit ist die Flächenkomponente 20 des vorliegenden Ausführungsbeispiels in dem Bereich des klebstofffreien Bereichs bzw. zwischen den beiden Streifen lose und nicht über die Klebstoffschicht 51 mit dem Trägerband 18 verbunden. Selbiges gilt, wie bereits beispielsweise anhand des in Figuren 5 und 6 dargestellten Ausführungsbeispiels erläutert, für die Bergspitzen bzw. Bergmitten 32.

Es versteht sich, dass ggf. der in Öffnungsvorzugsrichtung 60 liegende Rand der Klebstoffschicht 51 bzw. der Klebefläche 50 bis zu den Bergmitten 32 oder sogar darüber hinaus reichen kann, wenn dieses sinnvoll erscheint. Ebenso ist der andere Rand des in Öffnungsvorzugsrichtung 60 liegenden Streifens der Klebstoffschicht 51 hinsichtlich seiner genauen Position variierbar, um das jeweilige Verschlussband 10 hinsichtlich seiner Halteigenschaften anpassen zu können.

Es versteht sich, dass die Flächenkomponente 20 auf Höhe des klebstofffreien Bereichs auch mit einer Trägerbahnklebstoffschicht 58 mit dem Trägerband 18 verbunden sein kann.

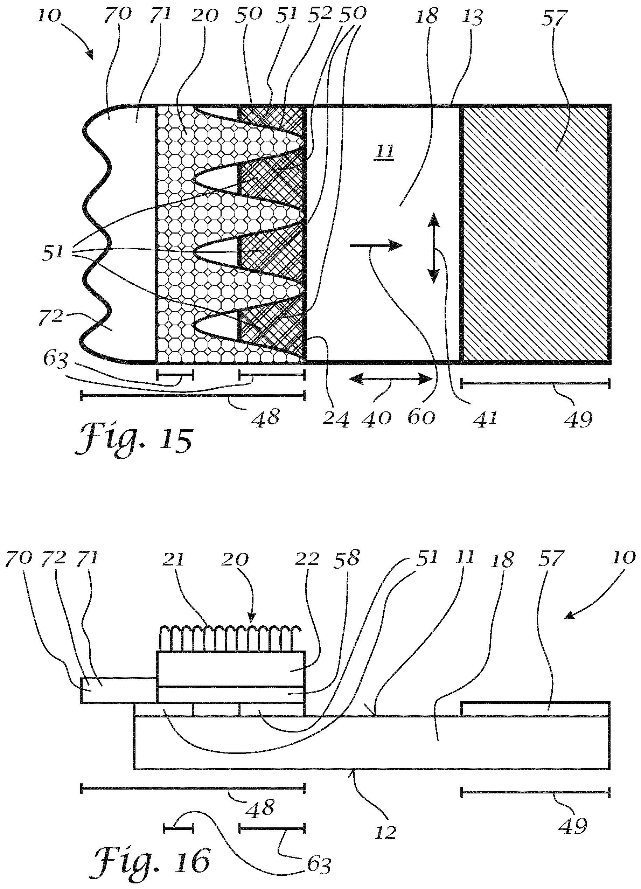

In einem weiteren Ausführungsbeispiel nach den Figuren 15 und 16 ist ein Verschlussband 10 vorgesehen, welches im Wesentlichen dem Verschluss nach den Figuren 13 und 14 entspricht, wobei die Klebefläche 50 und auch die Klebstoffschicht 51 bis zu Bergen 31 reicht. Somit liegt kein loser Bereich an den Spitzen der Berge 31 vor. Auch sind die bereits vorstehenden Variationen hinsichtlich des in Öffnungsvorzugsrichtung 60 angeordneten Streifens der Klebstoffschicht 51 denkbar.

Darüber hinaus ist bei diesem Ausführungsbeispiel eine Trägerbahnklebstoffschicht 58 vorgesehen, welche sicherstellt, dass keine losen Bereiche der Flächenkomponente 20 an Stellen zu finden sind, an denen kein Streifen der Klebstoffschicht 51 zu finden ist. Durch die Breite der Trägerbahnklebstoffschicht 58 sind auch hier Anpassungen hinsichtlich etwaiger loser Bereiche3 der Flächenkomponente 20 möglich.

Es versteht sich, dass ggf. auch die Klebstoffschicht 51 entsprechend gewellt bzw. mit Ausnehmungen versehen sein kann, um die bei vorliegenden Ausführungsbeispielen vorgesehen Aufgaben der Trägerbahnklebstoffschicht 58 zu übernehmen.

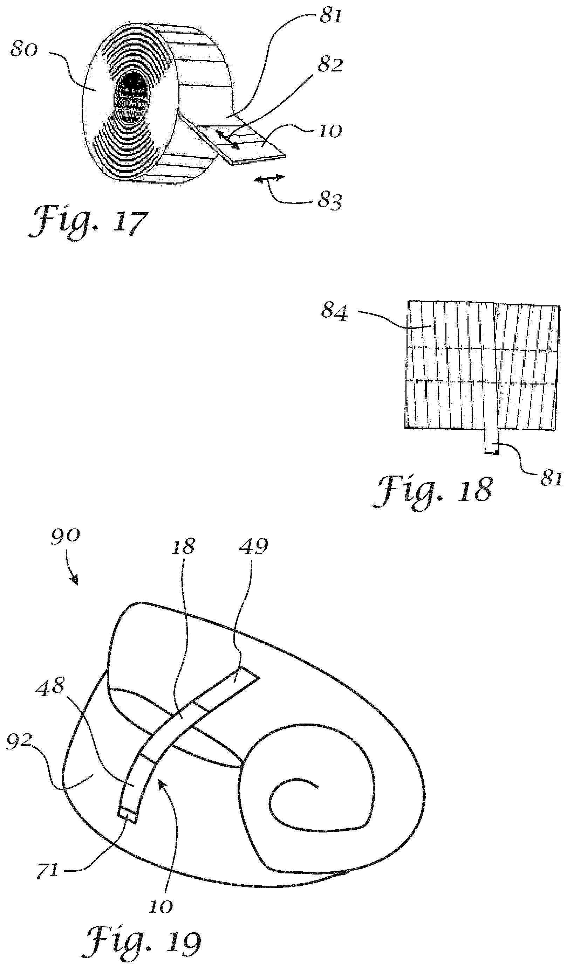

Das Verschlussband 10 kann, wie beispielhaft in der Figur 17 dargestellt, in Form einer Halbzeugrolle 80 bereitgestellt werden, bei der das Verschlussband 10 als Halbzeugbandmaterial 81 aufgerollt ist und von der Halbzeugrolle 80 in eine Maschinenrichtung 82 abrollbar ist. Somit kann durch ein Trennen in Querrichtung 83, welche senkrecht zur Maschinenrichtung 82 liegt, aus dem Halbzeugbandmaterial 81 ein Verschlussband 10 abgetrennt werden.

Ein solches Halbzeugbandmaterial 81 kann auch zu einer Halbzeugspule 84 aufgerollt sein, wie es schematisch in der Figur 18 dargestellt ist, indem die einzelnen Windungen nicht übereinander, sondern kreuzgewickelt abgelegt werden.

Das Verschlussband 10 kann bei Hygieneartikeln, wie beispielsweise einer Windel 90 zum Einsatz kommen.

Das Verschlussband 10 umfasst ein Trägerband 18 mit einem Benutzerbereich 48 und einem Permanentbereich 49 sowie einen Fingerlift 71 und ist mit seinem Permanentbereich 49 permanent an der Windel 90 befestigt. Dieser Bereich dient der permanenten Befestigung und ist nicht zum Ablösen vorgesehen. Bei einer theoretisch so starken Belastung des Verschlussbandes 10 über den Permanentbereich 49 könnte der Permanentbereich 49 allenfalls unter Überschreitung der mechanischen Höchstlast unter Zerstörung des Verschlussbandes 10 oder des Windelmaterials abgelöst werden.

Der Benutzerbereich 48 des Verschlussbandes 10 lässt sich mit der Windel 90 verbinden, wobei diese Verbindung als lösbare Verbindung vorgesehen ist. Beispielsweise soll das Verschlussband 10 in der Lage sein, eine Windel während der Benutzung in seinem Benutzungszustand zu belassen, wobei der Benutzerbereich mit der Windel 90 verbunden ist und diesen Zustand hält. Beim Wechseln der Windel 90 soll das Verschlussband 10 jedoch über den Benutzerbereich 48 gelöst und anschließend wieder zur Entsorgung mit der Windel 90 verbunden werden können, damit die gebrauchte Windel 90 im geschlossenen Zustand aus hygienischen Gründen entsorgt werden kann. Hierbei ist für den Benutzerbereich 48 ein zerstörungsfreies Lösen und Befestigen des Verschlussbandes 10 von der Windel 90 vorgesehen.

Der Benutzerbereich 48 des Verschlussbandes 10 dient hierbei als erste Flächenkomponente 20 eines zweikomponentigen mechanischen Flächenverschlusssystems und die Außenseite der Windel 90 selbst oder zumindest bestimmte Bereiche der Windel 90 als entsprechend zweite Flächenkomponente 92 des mechanischen zweikomponentigen Flächenverschlusssystems. Auf diese Weise werden die beiden Komponenten 20, 92 des Flächenverschlusssystems miteinander in Verbindung gebracht und verschließen entsprechend die Windel 90.

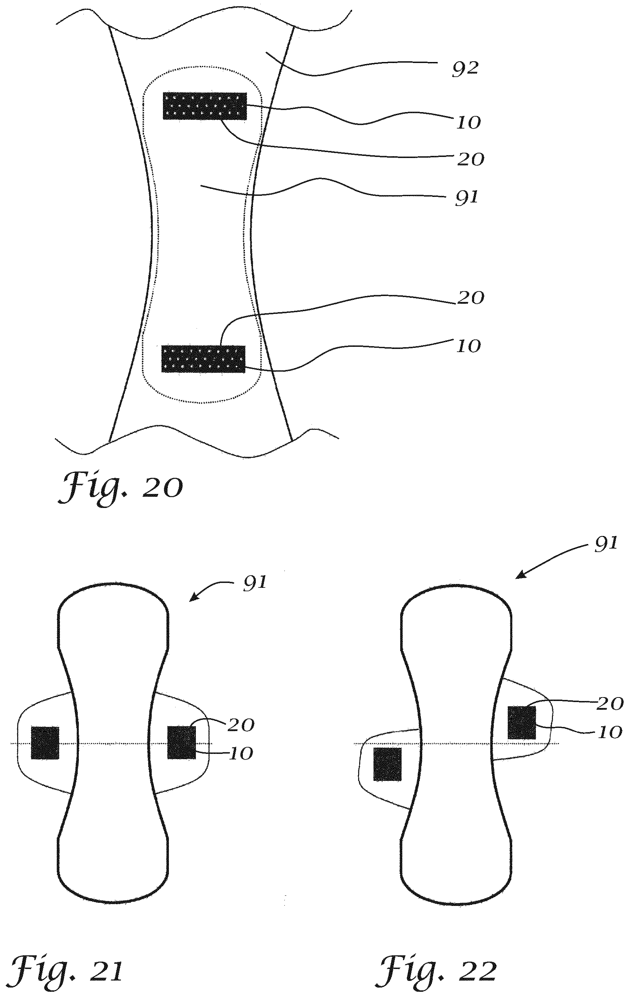

Bei einem weiteren Ausführungsbeispiel, wie es in der Figur 20 dargestellt ist, kann das Verschlussband 10 bei einer Damenbinde 91 verwendet werden und dazu dienen, die Damenbinde beispielsweise mit der Kleidung einer Trägerin zu verbinden oder in einer Entsorgungsposition aufzurollen und zu verschließen.

Das vorliegende Ausführungsbeispiel zeigt eine entsprechende Damenbinde 91 im eingelegten Zustand, wobei das Trägerband auf der Damenbinde 91 selbst angeordnet ist. Das Verschlussband 10 stellt hierbei eine erste Flächenkomponente 20 dar und im vorliegenden Ausführungsbeispiel stellt der Slip oder sonstige Kleidung, in denen die Damenbinde 91 eingelegt ist, die entsprechend zweite Flächenkomponente 92 dar. In einer Entsorgungskonfiguration können auch Seiten der Damenbinde entsprechend als zweite Flächenkomponente dienen.

Auch kann das Verschlussband 10 bei einer Damenbinde 91 verwendet werden, welche zwei parallele Flügelverschlüsse aufweist. Hierbei kann das Verschlussband 10 auf dem entsprechenden parallelen Flügelverschlüssen angeordnet sein und dient dann als erste Flächenkomponente 20, wie es schematisch in der Figur 21 dargestellt ist.

Wie beispielsweise in der Figur 22 dargestellt, kann die Damenbinde 91 auch versetzte Flügelverschlüsse aufweisen, wobei auch wieder Verschlussband 10 auf den versetzten Flügelverschlüssen als erste Flächenkomponente 20 angeordnet sein kann.

Es versteht sich, dass ein Verschlussband 10 nach der vorliegenden Erfindung auch für sämtliche andere Hygieneartikel eingesetzt werden kann, bei denen der Hygieneartikel selbst oder zumindest einen Bereich des Hygieneartikels wieder verschlossen werden soll und eine entsprechende lösbare Verbindung gewünscht ist. Besonders bevorzugt kann ein Verschlussband 10 bei Hygieneartikeln eingesetzt werden, bei denen durch den Schließbereich bzw. durch das Verschlussband 10 kein harter bzw. steifer Bereich entstehen sollte, da er aufgrund des Einsatzgebietes unangenehm für Anwender sein könnte.

Die vorgesehene Ausgestaltung des Verschlussbandes 10 sorgt für eine gute Verschlusskraft bei gleichzeitig hoher Flexibilität. Eine solche ist auch in dem vorliegenden Ausführungsbeispiel der Damenbinde 91 gefordert, da ein steifer Bereich des Verschlussbandes 10 ein unangenehmes Gefühl für die die Damenbinde 91 verwendende Person auslöst. Die Damenbinde 91 und der Gesamtbereich der Damenbinde 91 sollte für die anwendende Dame möglichst komfortable weich ausgestaltet sein. Durch die flexible Ausgestaltung des Verschlussbandes 10 schmiegt sich das Verschlussband 10 besser an die Damenbinde 91 und der beim Tragen ausbildenden Form an.

Ein her vorgestelltes Verschlussband 10 kann jedoch auch in sämtlichen anderen Bereichen als bei Hygieneartikeln eingesetzt werden, wo eine hohe Flexibilität des Verschlussbandes 10 bei gleichzeitig guter Haftung wünschenswert ist.

Beispielsweise wäre ein Einsatz des erfindungsgemäßen Verschlussbandes 10 bei Kleidungsstücken denkbar. So neigen beispielsweise Regenjacken oder anderen jackenartige Kleidungsstücke, welche sich zusätzlich zu einem Reisverschluss durch ein Verschlussband bzw. ein Klettverschluss abdichtend verschließen lassen dazu, dass eben dieses Verschlussband 10 für eine Versteifung des Kleidungsstücks im Bereich des Verschlussbandes 10 sorgt.

Auch die Verwendung des Verschlussbandes 10 beispielsweise bei Schutz,- OP- oder Besucherkitteln kann sich als besonders vorteilhaft erweisen.

Der Einsatz des hier vorgestellten Verschlussbandes 10 würde durch seine hohe Flexibilität bei gleichzeitig guter Verschlusskraft dem versteiften Verschluss entgegenwirken und einen entsprechend anschmiegsameren weniger steifen und flexibleren Verschluss bereitstellen können.

### Bezugszeichenliste:

- 10: Verschlussband
- 11: Befestigungsseite
- 12: Rückseite
- 13: Verschlussbandumgrenzung
- 14: Verschlussbandgrenze
- 15: Verschlussbandgrenze
- 16: Verschlussbandgrenze
- 17: Verschlussbandgrenze
- 18: Trägerband

- 20: erste Flächenkomponente
- 21: Haken
- 22: Trägerbahn
- 23: Flächenkomponentenrand
- 24: variierender Randbereich
- 25: Randbereich
- 26: Randbereich
- 27: Randbereich
- 29: Flächenpunkt der ersten Flächenkomponente 20

- 30: Randbereichsmittenlinie
- 31: Berg
- 32: Bergmitte
- 33: Abstand benachbarter Bergmitten 32
- 34: Tal
- 35: Talmitte
- 36: Abstand benachbarter Talmitten 35
- 37: Variationsspanne
- 38: Schnittpunkt zwischen Randbereichsmittenlinie 30 und Flächenkomponentenrand 23
- 39: Winkel zwischen Randbereichsmittenlinie 30 und Flächenkomponentenrand 23

- 40: Breite
- 41: Höhe
- 48: Benutzerbereich
- 49: Permanentbereich

- 50: Klebefläche
- 51: Klebstoffschicht
- 52: loser Bereich
- 57: Klebstoffschicht des Permanentbereichs 49
- 58: Trägerbahnklebstoffschicht
- 59: Flächenpunkt der Klebefläche 50

- 60: Öffnungsvorzugsrichtung
- 61: Flächenüberdeckung einer Höhe 31
- 62: Flächenüberdeckung einer Tiefe 34
- 63: Überlappung zwischen erster Flächenkomponente und Klebstoffschicht
- 64: 80%-Durchgang
- 65: Abstand zweier 80%-Durchgänge 64
- 66: Übergang
- 67: Erstreckung des Übergangs 66 entlang der Abszisse

- 70: Anfasser
- 71: Fingerlift
- 72: an- und aufgesetztes Griffband

- 80: Halbzeugrolle
- 81: Halbzeugbandmaterial
- 82: Maschinenrichtung
- 83: Querrichtung
- 84: Halbzeugspule

- 90: Windel
- 91: Damenbinde
- 92: zweite Flächenkomponente

## Patentansprüche

1. Verschlussband (10) mit einer Befestigungsseite (11), an welcher eine erste Flächenkomponente (20) eines zweikomponentigen mechanischen Flächenverschlusssystems befindlich ist, mit einer der Befestigungsseite (11) abgewandten Rückseite (12) und mit einer sich parallel zur Befestigungsseite (11) und zur Rückseite (12) erstreckenden Breite (40) und einer sich ebenfalls parallel zur Befestigungsseite (11) und zur Rückseite (12) erstreckenden und senkrecht zur Breite (40) ausgerichteten Höhe (41) sowie mit einer umlaufenden Verschlussbandumgrenzung (13), die zwei sich parallel zur Breite (40) erstreckende Verschlussbandgrenzen (16, 17) und zwei sich senkrecht zur Breite (40) erstreckende Verschlussbandgrenzen (14, 15) umfasst, **dadurch gekennzeichnet, dass** parallel zur Breite (40) eine Öffnungsvorzugsrichtung (60) definiert ist und an der Befestigungsseite (11) ergänzend zu der ersten Flächenkomponente (20) des mechanischen Flächenverschlusssystems eine zusammenhängende oder nicht zusammenhängende Klebefläche (50) vorgesehen ist und dass von jedem Flächenpunkt (59) der Klebefläche (50) in Richtung der Öffnungsvorzugsrichtung (60) kein Flächenpunkt (29) der ersten Flächenkomponente (20) angeordnet ist.

2. Verschlussband (10) nach Anspruch 1, **dadurch gekennzeichnet, dass** sich die Klebefläche (50) in Richtung der Öffnungsvorzugsrichtung (60) unmittelbar an die Flächenkomponente (20) anschließt.

3. Verschlussband (10) nach Anspruch 1 oder 2, **dadurch gekennzeichnet, dass** die Flächenkomponente (20) einen Flächenkomponentenrand (23) mit einem in Öffnungsvorzugsrichtung (60) weisenden Randbereich (24) aufweist, der vorzugsweise gekrümmt oder senkrecht zur Öffnungsvorzugsrichtung (60) geneigt ist und/oder variiert.

4. Verschlussband (10) nach einem der Ansprüche 1 bis 3, **dadurch gekennzeichnet, dass** die Variation periodisch, vorzugsweise wellen- und/oder sinusförmig, ausgebildet ist, wobei die Variation insbesondere Berge (31) und Täler (34) aufweist.

5. Verschlussband (10) nach Anspruch 4, **dadurch gekennzeichnet, dass** zwischen einer Talmitte (35) und der Klebefläche (50) ein klebstofffreier Bereich angeordnet ist und/oder dass sich die Klebefläche (50) in Öffnungsvorzugsrichtung (60) bis zu einer Bergmitte (32) erstreckt.

6. Verschlussband (10) nach einem der Ansprüche 1 bis 4, **dadurch gekennzeichnet, dass** sich die Flächenkomponente (20) in Richtung der Öffnungsvorzugsrichtung (60) weiter erstreckt als die Klebefläche (50).

7. Verschlussband (10) nach Anspruch 6, **dadurch gekennzeichnet, dass** der lose Bereich (52) sich von der Bergmitte (32) ausgehend bis zum Beginn der Klebefläche (50) erstreckt.

8. Verschlussband (10) nach einem der Ansprüche 1 bis 7, **dadurch gekennzeichnet, dass** die Öffnungsvorzugsrichtung (60) durch das Anordnen eines Anfassers (70) an dem Verschlussband (10) definiert ist.

9. Verschlussband (10) nach Anspruch 8, **dadurch gekennzeichnet, dass** der Anfasser (70) an der entgegen der Öffnungsvorzugsrichtung (60) angeordneten Verschlussbandgrenze (15) angeordnet ist.

10. Verschlussband (10) nach Anspruch 8 oder 9, **dadurch gekennzeichnet, dass** der Anfasser (70) als Fingerlift (71), der vorzugsweise ein an- oder aufgesetztes Griffband (72) und/oder eine gewellte Verschlussbandgrenze (15) umfasst, ausgestaltet ist.

11. Verschlussband (10) nach einem der Ansprüche 1 bis 10, **dadurch gekennzeichnet, dass** die erste Flächenkomponente (20) ein Hakenmaterial mit von einer Trägerbahn (22) getragenen Haken (21) oder dass die erste Flächenkomponente (20) ein Schlaufenmaterial ist.

12. Verschlussband (10) nach einem der Ansprüche 1 bis 11, **dadurch gekennzeichnet, dass** die erste Flächenkomponente (20) als zusammenhängendes Gebiet, insbesondere als einfach zusammenhängendes Gebiet, ausgebildet ist.

13. Verschlussband (10) nach einem der Ansprüche 1 bis 12, **dadurch gekennzeichnet, dass** das Verschlussband (10) ein Trägerband (18) umfasst, welches die erste Flächenkomponente (20) sowie ggf. eine die Klebefläche (50) bildende Klebstoffschicht (51) und/oder den Anfasser (70) trägt.

14. Verschlussband (10) nach Anspruch 13, **dadurch gekennzeichnet, dass** die Klebstoffschicht die Flächenkomponente (50) an dem Trägerband (18) befestigt.

15. Verschlussband (10) nach einem der Ansprüche 1 bis 14, **dadurch gekennzeichnet, dass** das Verschlussband (10) einen Permanentbereich (49) zur permanenten Befestigung an einem mit dem Verschlussband (10) zu verschließenden Gegenstand, wie einem Kleidungsstück oder einem Hygieneartikel, und einen Benutzerbereich (48) zur lösbaren Befestigung an dem Gegenstand aufweist, wobei der Permanentbereich (49) und der Benutzerbereich (48) vorzugsweise senkrecht zur Befestigungs- und zur Rückseite (12) überschneidend oder voneinander beabstandet angeordnet sind.

16. Verschlussband (10) nach einem der Ansprüche 1 bis 15, **dadurch gekennzeichnet, dass** die erste Flächenkomponente (20) einen Flächenkomponentenrand (23) aufweist, der wenigstens einen in der Höhe (41) verlaufenden variierenden Randbereich (24) aufweist, der in seinem Verlauf entlang der Höhe (41) in Richtung der Breite (40) um eine Randbereichsmittenlinie (30), die Schnittpunkte (38) zwischen dem Flächenkomponentenrand (23) und der Randbereichsmittenlinie (30) trägt, variiert, wobei die Variation in Richtung der Höhe (41) Berge (31) und Täler (34) aufweist und wobei der Variation in dem variierenden Randbereich (24) ein Mittenvariationswert zugeordnet werden kann, welcher von der absoluten Abweichung von Variationsspannen (37) zwischen Berge (31) und Täler (34) zu der Randbereichsmittenlinie (30) abhängig ist, und wobei der Mittenvariationswert zumindest so groß wie die Hälfte des mittleren Abstandes (33), insbesondere zumindest so groß wie der 0,38-fache, insbesondere der 0,39-fache, mittlere Abstand (33), parallel zur Höhe (41) zwischen zwei benachbarten Bergmitten (32) und/oder zwei benachbarten Talmitten (35) ist.

17. Verschlussband (10) nach einem der Ansprüche 1 bis 16, **dadurch gekennzeichnet, dass** die erste Flächenkomponente (20) einen Flächenkomponentenrand (23) aufweist, der wenigstens einen in der Höhe (41) verlaufenden variierenden Randbereich (24) aufweist, der in seinem Verlauf entlang der Höhe (41) in Richtung der Breite (40) um eine Randbereichsmittenlinie (30), die Schnittpunkte (38) zwischen dem Flächenkomponentenrand (23) und der Randbereichsmittenlinie (30) trägt, variiert, wobei die Variation in Richtung der Höhe (41) Berge (31) und Täler (34) aufweist und wobei die Zahl der Berge (31) 2 pro 1 cm Erstreckung des Randbereichs (25) und/oder die Zahl der Täler (34) 2 pro 1 cm Erstreckung des Randbereichs (25) übersteigt, wobei insbesondere die Zahl der Berge (31) bzw. Täler (34) 2,3, vorzugsweise 2,4, pro 1 cm Erstreckung des Randbereichs (25) übersteigt und/oder dass die Zahl der Berge (31) bzw. Täler (34) 2 pro 0,9 cm, vorzugsweise pro 0,8 cm, Erstreckung des Randbereichs (25) übersteigt.

18. Verschlussband (10) nach einem der Ansprüche 1 bis 17, **dadurch gekennzeichnet, dass** die Summe aus dem Absolutwert der Variationsspanne (37) einer Bergmitte (32) und dem Absolutwert der Variationsspanne (37) einer zu dieser Bergmitte (32) benachbarten Talmitte (35) zumindest so groß ist, insbesondere zumindest 1,2-mal, vorzugsweise 1,5-mal, wie der doppelte Abstand parallel zur Höhe (41) zwischen dieser Talmitte (35) und dieser Bergmitte (32) und/oder die mittleren Abstände der Bergmitten (32) und/oder die mittleren Abstände der Talmitten.

19. Verschlussband (10) nach einem der Ansprüche 1 bis 18, **dadurch gekennzeichnet, dass** die Randbereichsmittenlinie (30) aus dem Mittelwert des variierenden Randbereichs (24) in Bezug auf die Höhe (41) bzw. senkrecht zur Breite (40) bestimmt ist und/oder dass die Variationsspanne (37) jeweils als der Abstand zwischen Randbereichsmittenlinie (30) und dem variierenden Randbereich (24) bestimmt ist und/oder dass der Mittenvariationswert der Mittelwert der absoluten Variationsspannen (37) über der Erstreckung des variierenden Randbereichs (24) ist.

20. Halbzeugbandmaterial (81) mit einer Maschinenrichtung (81) und einer senkrecht hierzu angeordneten Querrichtung (83) zur Herstellung eines Verschlussbands (10) nach einem der vorstehenden Ansprüche mittels Trennen des Halbzeugbandmaterials (81) in Querrichtung (83), wobei die Maschinenrichtung (81) der Höhe (41) und die Querrichtung (83) der Breite (40) des Verschlussbands (10) entsprechen.

21. Verfahren zur Abstimmung der Klebekräfte bei einem Verschlussband (10) nach einem der Ansprüche 1 bis 19 durch Variation der Überlappung (63) zwischen der ersten Flächenkomponente (20) und einer die Klebfläche (50) bildenden unter der ersten Flächenkomponente (20) angeordneten Klebstoffschicht (51).

## Claims

1. Fastening tape (10) having an attachment side (11), on which a first surface component (20) of a two-component mechanical surface fastening system is located, having a rear side (12) facing away from the attachment side (11), and having a width (40) extending parallel to the attachment side (11) and to the rear side (12), and a height (41) likewise extending parallel to the attachment side (11) and to the rear side (12) and oriented perpendicular to the width (40), and having a circumferential fastening tape boundary (13), which comprises two fastening tape edges (16, 17) extending parallel to the width (40) and two fastening tape edges (14, 15) extending perpendicular to the width (40), **characterized in that** an opening preferred direction (60) is defined parallel to the width (40), and an adhesive surface (50), which is continuous or non-continuous, is provided on the attachment side (11) in addition to the first surface component (20) of the mechanical surface fastening system, and **in that**, from each surface point (59) of the adhesive surface (50), no surface point (29) of the first surface component (20) is arranged in the opening preferred direction (60).

2. Fastening tape (10) according to claim 1, **characterized in that** the adhesive surface (50) directly adjoins the surface component (20) in the opening preferred direction (60).

3. Fastening tape (10) according to claim 1 or 2, **characterized in that** the surface component (20) has a surface component edge (23) with an edge region (24) facing in the opening preferred direction (60), which edge region is preferably curved or inclined perpendicular to the opening preferred direction (60) and/or varies.

4. Fastening tape (10) according to any one of claims 1 to 3, **characterized in that** the variation is periodic, preferably wave-shaped and/or sinusoidal, wherein the variation in particular has peaks (31) and valleys (34).

5. Fastening tape (10) according to claim 4, **characterized in that** an adhesive-free region is arranged between a valley center (35) and the adhesive surface (50), and/or **in that** the adhesive surface (50) extends in the opening preferred direction (60) as far as a peak center (32).

6. Fastening tape (10) according to any one of claims 1 to 4, **characterized in that** the surface component (20) extends farther in the opening preferred direction (60) than the adhesive surface (50).

7. Fastening tape (10) according to claim 6, **characterized in that** the loose region (52) extends from the peak center (32) as far as the beginning of the adhesive surface (50).

8. Fastening tape (10) according to any one of claims 1 to 7, **characterized in that** the opening preferred direction (60) is defined by arranging a gripping element (70) on the fastening tape (10).

9. Fastening tape (10) according to claim 8, **characterized in that** the gripping element (70) is arranged on the fastening tape edge (15) arranged opposite the opening preferred direction (60).

10. Fastening tape (10) according to claim 8 or 9, **characterized in that** the gripping element (70) is configured as a fingerlift (71), which preferably comprises an attached or applied grip tape (72) and/or a corrugated fastening tape edge (15).

11. Fastening tape (10) according to any one of claims 1 to 10, **characterized in that** the first surface component (20) is a hook material having hooks (21) carried by a carrier web (22), or **in that** the first surface component (20) is a loop material.

12. Fastening tape (10) according to any one of claims 1 to 11, **characterized in that** the first surface component (20) is formed as a connected region, in particular as a simply connected region.

13. Fastening tape (10) according to any one of claims 1 to 12, **characterized in that** the fastening tape (10) comprises a carrier tape (18), which carries the first surface component (20) and, if applicable, an adhesive layer (51) forming the adhesive surface (50), and/or the gripping element (70).

14. Fastening tape (10) according to claim 13, **characterized in that** the adhesive layer attaches the surface component ( 20 ) to the carrier tape (18).

15. Fastening tape (10) according to any one of claims 1 to 14, **characterized in that** the fastening tape (10) has a permanent region (49) for permanent attachment to an article to be closed by the fastening tape (10), such as a garment or a hygiene article, and a user region (48) for releasable attachment to the article, wherein the permanent region (49) and the user region (48) are preferably arranged overlapping or spaced apart from one another perpendicular to the attachment side (11) and to the rear side (12).

16. Fastening tape (10) according to any one of claims 1 to 15, **characterized in that** the first surface component (20) has a surface component edge (23), which has at least one varying edge region (24) extending in the height (41), which varying edge region varies in its course along the height (41) in the direction of the width (40) about an edge region center line (30), which carries intersection points (38) between the surface component edge (23) and the edge region center line (30), wherein the variation has peaks (31) and valleys (34) in the direction of the height (41), and wherein a mean variation value can be assigned to the variation in the varying edge region (24), which mean variation value depends on the absolute deviation of variation spans (37) between peaks (31) and valleys (34) from the edge region center line (30), and wherein the mean variation value is at least as large as half of the mean distance (33), in particular at least as large as 0.38 times, in particular 0.39 times, the mean distance (33), parallel to the height (41), between two adjacent peak centers (32) and/or two adjacent valley centers (35).

17. Fastening tape (10) according to any one of claims 1 to 16, **characterized in that** the first surface component (20) has a surface component edge (23), which has at least one varying edge region (24) extending in the height (41), which varying edge region varies in its course along the height (41) in the direction of the width (40) about an edge region center line (30), which carries intersection points (38) between the surface component edge (23) and the edge region center line (30), wherein the variation has peaks (31) and valleys (34) in the direction of the height (41), and wherein the number of peaks (31) exceeds 2 per 1 cm extent of the edge region (25) and/or the number of valleys (34) exceeds 2 per 1 cm extent of the edge region (25), wherein in particular the number of peaks (31) or valleys (34) exceeds 2.3, preferably 2.4, per 1 cm extent of the edge region (25), and/or **in that** the number of peaks (31) or valleys (34) exceeds 2 per 0.9 cm, preferably per 0.8 cm, extent of the edge region (25).

18. Fastening tape (10) according to any one of claims 1 to 17, **characterized in that** the sum of the absolute value of the variation span (37) of a peak center (32) and the absolute value of the variation span (37) of a valley center (35) adjacent to this peak center (32) is at least as large as, in particular at least 1.2 times, preferably 1.5 times, twice the distance parallel to the height (41) between this valley center (35) and this peak center (32) and/or the mean distances of the peak centers (32) and/or the mean distances of the valley centers.

19. Fastening tape (10) according to any one of claims 1 to 18, **characterized in that** the edge region center line (30) is determined from the mean value of the varying edge region (24) with respect to the height (41) or perpendicular to the width (40), and/or **in that** the variation span (37) is in each case determined as the distance between the edge region center line (30) and the varying edge region (24), and/or **in that** the mean variation value is the mean value of the absolute variation spans (37) over the extent of the varying edge region (24).

20. Semi-finished tape material (81) having a machine direction (81) and a transverse direction (83) arranged perpendicular thereto, for producing a fastening tape (10) according to any one of the preceding claims by separating the semi-finished tape material (81) in the transverse direction (83), wherein the machine direction (81) corresponds to the height (41), and the transverse direction (83) corresponds to the width (40), of the fastening tape (10).

21. Method for adjusting the adhesive forces in a fastening tape (10) according to any one of claims 1 to 19 by varying the overlap (63) between the first surface component (20) and an adhesive layer (51), which forms the adhesive surface (50) and is arranged beneath the first surface component (20).

## Revendications

1. Ruban de fermeture (10) comprenant un côté de fixation (11), sur lequel se trouve un premier composant surfacique (20) d'un système de fermeture surfacique mécanique à deux composants, comprenant un côté arrière (12) détourné du côté de fixation (11), et comprenant une largeur (40) s'étendant parallèlement au côté de fixation (11) et au côté arrière (12), ainsi qu'une hauteur (41) s'étendant également parallèlement au côté de fixation (11) et au côté arrière (12) et orientée perpendiculairement à la largeur (40), ainsi qu'une délimitation périphérique de ruban de fermeture (13), qui comprend deux limites de ruban de fermeture (16, 17) s'étendant parallèlement à la largeur (40) et deux limites de ruban de fermeture (14, 15) s'étendant perpendiculairement à la largeur (40), **caractérisé en ce qu'**une direction préférentielle d'ouverture (60) est définie parallèlement à la largeur (40), et qu'une surface adhésive (50), continue ou non continue, est prévue sur le côté de fixation (11), en complément du premier composant surfacique (20) du système de fermeture surfacique mécanique, et **en ce qu'**à partir de chaque point surfacique (59) de la surface adhésive (50), aucun point surfacique (29) du premier composant surfacique (20) n'est disposé dans la direction préférentielle d'ouverture (60).

2. Ruban de fermeture (10) selon la revendication 1, **caractérisé en ce que** la surface adhésive (50) jouxte directement le composant surfacique (20) dans la direction préférentielle d'ouverture (60).

3. Ruban de fermeture (10) selon la revendication 1 ou 2, **caractérisé en ce que** le composant surfacique (20) présente un bord de composant surfacique (23) comprenant une région de bord (24) orientée dans la direction préférentielle d'ouverture (60), laquelle est de préférence courbée ou inclinée perpendiculairement à la direction préférentielle d'ouverture (60) et/ou varie.

4. Ruban de fermeture (10) selon l'une des revendications 1 à 3, **caractérisé en ce que** la variation est périodique, de préférence ondulée et/ou sinusoïdale, la variation présentant en particulier des pics (31) et des creux (34).

5. Ruban de fermeture (10) selon la revendication 4, **caractérisé en ce qu'**une région exempte d'adhésif est disposée entre un centre de creux (35) et la surface adhésive (50), et/ou **en ce que** la surface adhésive (50) s'étend dans la direction préférentielle d'ouverture (60) jusqu'à un centre de pic (32).

6. Ruban de fermeture (10) selon l'une des revendications 1 à 4, **caractérisé en ce que** le composant surfacique (20) s'étend dans la direction préférentielle d'ouverture (60) plus loin que la surface adhésive (50).

7. Ruban de fermeture (10) selon la revendication 6, **caractérisé en ce que** la région libre (52) s'étend depuis le centre de pic (32) jusqu'au début de la surface adhésive (50).

8. Ruban de fermeture (10) selon l'une des revendications 1 à 7, **caractérisé en ce que** la direction préférentielle d'ouverture (60) est définie par la disposition d'un élément de préhension (70) sur le ruban de fermeture (10).

9. Ruban de fermeture (10) selon la revendication 8, **caractérisé en ce que** l'élément de préhension (70) est disposé sur la limite de ruban de fermeture (15) située à l'opposé de la direction préférentielle d'ouverture (60).

10. Ruban de fermeture (10) selon la revendication 8 ou 9, **caractérisé en ce que** l'élément de préhension (70) est configuré comme une languette de préhension (71), qui comprend de préférence une bande de préhension rapportée ou appliquée (72) et/ou une limite de ruban de fermeture ondulée (15).

11. Ruban de fermeture (10) selon l'une des revendications 1 à 10, **caractérisé en ce que** le premier composant surfacique (20) est un matériau à crochets comportant des crochets (21) portés par une bande support (22), ou **en ce que** le premier composant surfacique (20) est un matériau à boucles.

12. Ruban de fermeture (10) selon l'une des revendications 1 à 11, **caractérisé en ce que** le premier composant surfacique (20) est réalisé sous forme d'une région connexe, en particulier sous forme d'une région simplement connexe.

13. Ruban de fermeture (10) selon l'une des revendications 1 à 12, **caractérisé en ce que** le ruban de fermeture (10) comprend une bande porteuse (18), qui porte le premier composant surfacique (20) ainsi que, le cas échéant, une couche adhésive (51) formant la surface adhésive (50), et/ou l'élément de préhension (70).

14. Ruban de fermeture (10) selon la revendication 13, **caractérisé en ce que** la couche adhésive fixe le composant surfacique (20) à la bande porteuse (18).

15. Ruban de fermeture (10) selon l'une des revendications 1 à 14, **caractérisé en ce que** le ruban de fermeture (10) présente une région permanente (49) destinée à la fixation permanente à un objet à fermer au moyen du ruban de fermeture (10), tel qu'un vêtement ou un article d'hygiène, et une région d'utilisation (48) destinée à la fixation amovible à l'objet, la région permanente (49) et la région d'utilisation (48) étant de préférence disposées en chevauchement ou espacées l'une de l'autre perpendiculairement au côté de fixation et au côté arrière (12).

16. Ruban de fermeture (10) selon l'une des revendications 1 à 15, **caractérisé en ce que** le premier composant surfacique (20) présente un bord de composant surfacique (23), qui présente au moins une région de bord variable (24) s'étendant dans la hauteur (41), laquelle varie, dans son tracé le long de la hauteur (41), dans la direction de la largeur (40), autour d'une ligne médiane de région de bord (30), qui porte des points d'intersection (38) entre le bord de composant surfacique (23) et la ligne médiane de région de bord (30), la variation présentant des pics (31) et des creux (34) dans la direction de la hauteur (41), et une valeur moyenne de variation pouvant être attribuée à la variation dans la région de bord variable (24), laquelle valeur moyenne de variation dépend de l'écart absolu de portées de variation (37) entre pics (31) et creux (34) par rapport à la ligne médiane de région de bord (30), et la valeur moyenne de variation étant au moins aussi grande que la moitié de la distance moyenne (33), en particulier au moins aussi grande que 0,38 fois, en particulier 0,39 fois, la distance moyenne (33), parallèlement à la hauteur (41), entre deux centres de pic adjacents (32) et/ou deux centres de creux adjacents (35).

17. Ruban de fermeture (10) selon l'une des revendications 1 à 16, **caractérisé en ce que** le premier composant surfacique (20) présente un bord de composant surfacique (23), qui présente au moins une région de bord variable (24) s'étendant dans la hauteur (41), laquelle varie, dans son tracé le long de la hauteur (41), dans la direction de la largeur (40), autour d'une ligne médiane de région de bord (30), qui porte des points d'intersection (38) entre le bord de composant surfacique (23) et la ligne médiane de région de bord (30), la variation présentant des pics (31) et des creux (34) dans la direction de la hauteur (41), et le nombre de pics (31) dépassant 2 par 1 cm d'étendue de la région de bord (25) et/ou le nombre de creux (34) dépassant 2 par 1 cm d'étendue de la région de bord (25), le nombre de pics (31) ou de creux (34) dépassant notamment 2,3, de préférence 2,4, par 1 cm d'étendue de la région de bord (25), et/ou le nombre de pics (31) ou de creux (34) dépassant 2 par 0,9 cm, de préférence par 0,8 cm, d'étendue de la région de bord (25).

18. Ruban de fermeture (10) selon l'une des revendications 1 à 17, **caractérisé en ce que** la somme de la valeur absolue de la portée de variation (37) d'un centre de pic (32) et de la valeur absolue de la portée de variation (37) d'un centre de creux (35) adjacent à ce centre de pic (32) est au moins aussi grande que, en particulier au moins 1,2 fois, de préférence 1,5 fois, le double de la distance parallèle à la hauteur (41) entre ce centre de creux (35) et ce centre de pic (32) et/ou les distances moyennes des centres de pic (32) et/ou les distances moyennes des centres de creux.

19. Ruban de fermeture (10) selon l'une des revendications 1 à 18, **caractérisé en ce que** la ligne médiane de région de bord (30) est déterminée à partir de la valeur moyenne de la région de bord variable (24) par rapport à la hauteur (41) ou perpendiculairement à la largeur (40), et/ou **en ce que** la portée de variation (37) est déterminée respectivement comme la distance entre la ligne médiane de région de bord (30) et la région de bord variable (24), et/ou **en ce que** la valeur moyenne de variation est la valeur moyenne des portées de variation absolues (37) sur l'étendue de la région de bord variable (24).

20. Matériau semi-fini en bande (81) comprenant une direction machine (81) et une direction transversale (83) disposée perpendiculairement à celle-ci, pour la fabrication d'un ruban de fermeture (10) selon l'une des revendications précédentes au moyen d'une séparation du matériau semi-fini en bande (81) dans la direction transversale (83), la direction machine (81) correspondant à la hauteur (41) et la direction transversale (83) correspondant à la largeur (40) du ruban de fermeture (10).

21. Procédé d'ajustement des forces adhésives dans un ruban de fermeture (10) selon l'une des revendications 1 à 19 par variation du chevauchement (63) entre le premier composant surfacique (20) et une couche adhésive (51), formant la surface adhésive (50), disposée sous le premier composant surfacique (20).
